# EUROPEAN PATENT SPECIFICATION

(11) **EP 3 897 156 B1**
(45) Date of publication and mention of the grant of the patent: **09.07.2025**
(21) Application number: 19832625.8
(22) Date of filing: 17.12.2019
(51) Int. Cl.: A01N 63/20, A01P 5/00, C12R 1/20, C12N 1/20

(54) **MICROBACTERIUM ESTERAROMATICUM STRAIN, COMPOSITION COMPRISING THE SAME, AND USES THEREOF**
MIKROBAKTERIUM ESTERAROMATICUM-STAMM, ZUSAMMENSETZUNG DAMIT UND VERWENDUNGEN DAVON
SOUCHE DE MICROBACTERIUM ESTERAROMATICUM, COMPOSITION LA COMPRENANT ET SES UTILISATIONS

(30) Priority: 18.12.2018 EP 18382937
(43) Date of publication of application: 27.10.2021
(73) Proprietor: Futureco Bioscience, S.A., 08799 Olèrdola - Barcelona (ES)
(72) Inventor: LARA SÁNCHEZ, José Manuel, 08799 Olérdola (ES); FERNÁNDEZ CASTILLO, Carolina, 08799 Olérdola (ES)
(74) Representative: ZBM Patents - Zea, Barlocci & Markvardsen
(86) International application number: PCT/EP2019/085774
(87) International publication number: WO 2020/127364

(56) References cited:
- WO-A1-2008/049230
- WO-A1-2013/138398
- US-A1- 2014 056 853

## Description

This application claims the benefit of European Patent Application EP18382937.3 filed on December 18th, 2018.

### Technical Field

The present invention relates to the field of phytosanitary products. It provides a isolated strain of the microorganism *Microbacterium esteraromaticum,* compositions comprising it and their use in the biological control of diseases caused by parasitic nematodes in plants.

### Background art

Parasitic nematodes cause important plant losses of great economic importance around the world, being among the major limiting factors in field and plantation crop production. Parasitic nematodes include root-knot nematodes, e.g., the *Meloidogyne* genus; cyst nematodes, e.g., the *Globodera* genus; and root-lesion nematodes, e.g., the *Pratylenchus* genus.

The most commonly used nematode management strategy is chemical control but, although is effective, it may lead to soil pollution problems, affecting biodiversity and having a negative impact on human health. Over the last decades, researchers have tried to develop non-chemical and ecofriendly approaches. Some studies have evaluated the potential of plant extracts comprising rhizobacteria strains to inhibit parasitic nematodes, wherein it has been suggested that the nematicidal effect is also performed by biocidal substances released by the plant. In Sturz AV *et al.* (Sturz AV and Kimpinski J 2004 Plan and Soil 262:241-249) rhizobacterial antagonists of plant parasitic nematodes were identified in marigold extracts but their efficacy was restricted to the nematodes present in the soil and they were not effective against the parasitic nematodes within the root tissues.

WO 2013/138398 A1 and US 2014/056853 A1 disclose the use of a *Microbacterium* (*=Flavobacterium*) strain H492 (NRRL Accession No. B-50584), its whole cell broth and its cell-free supernatant for controlling parasitic nematodes in plants.

It is important to point out that the environmental and host conditions affect the biological activity of the bacteria strains, making their effectiveness generally variable and significantly lower than the reference chemical products.

In spite of the efforts made, there is a need for bacterial strains with nematicidal properties which overcome all or part of the limitations shown by the bacterial strains already known in the state of the art in controlling parasitic plant nematodes, specially being effective against the parasitic nematodes present in the root tissues.

### Summary of the invention

The inventors have surprisingly found one strain of the *Microbacterium esteraromaticum* species (hereinafter also referred as "bacterial strain B24" or "B24") which presents antagonist activity against plant-parasitic nematodes, such as root-knot nematodes and cyst nematodes, which performs its activity in the nematodes present in the plant (i.e.: root) and that exercises said activity in a wide range of soil pH.

As it is shown below, the strain of the invention has been tested in growth chamber and greenhouse trials and has demonstrated to be efficient, in controlling eggs and juvenile root-knot nematodes (RKNs) *Meloidogyne incognita* and *Meloidogyne javanica* and against the potato cyst nematodes (PCNs) *Globodera rostochiensis* and *Globodera pallida.*

It is remarkable that the *in vitro* efficacy of the strain of the invention against the tested nematodes, in terms of egg hatching inhibition, was similar to the one achieved using the reference nematicidal chemical in RKN nematodes (reference chemical was Fenamiphos) (Fig. 1 and 2). Surprisingly, the nematicidal effect of the strain of the invention *in vitro* against cysts of PCN was better than that of the reference chemical (Oxamyl) (example 5, figs. 7 and 8). This anti-nematode effect, in terms of nematode control, was also detected *in vivo,* measuring the final population of RKN eggs per plant and per gram of fresh root (RKN reproduction). This *in vivo* effect was achieved using the bacteria of the invention in several forms: as pellet (example 6.1), as Technical Grade Active Ingredient (TGAI) (example 6.2) and as formulated prototype (oil dispersion) (example 6.3). Surprisingly in cucumber plants *in vivo* the efficacy on final population and on reproduction was better than the reference chemical (see example 6.4).

From these experimental data it could be concluded that the bacterial strain of the present invention has a surprising effect controlling a broad range of nematodes, independently of their developmental stage and part of the plant. This is the first time that it is reported a strain of *M. esteraromaticum* with such particular effect.

In addition, to such remarkable anti-nematode activity profile, the inventors also found that the strain of the present invention was safe, as it is shown in example 7 when mice were fed with the strain of the invention as TGAI no pathogenicity was found.

Without being bound to any theory, the inventors believe that such nematicidal effect is due to the fact that the strain of the present invention contacts the host and releases certain enzymes (see example 1, wherein nematicidal effect is observed with the supernatant) and participates in the Induced Systemic Resistance (ISR) of the plant.

Furthermore, the strain of the invention has the ability to colonize and survive in soils with pH ranging from 5 to 10.

The pH range of soils, can range from ultra-acidic, which are soils that have pH lower than 3.5, to very strongly alkaline soils, which have pH higher than 9. The soil pH has a clear impact on plant nutrients availability. This is the reason why there are some plant species that can grow in some soils, and other that cannot, depending on their growth requirements. On the other hand, it is well-recognized by the skilled person in the art that environment conditions such as pH can negatively affect the viability/activity of a particular microorganism. The inventors have surprisingly found that the strain of the invention can survive at extreme pH values, between 5 and 10. This is a further valuable advantage because it means that the strain of the invention can exert its function in a wide range of plant crops.

Moreover, the inventors have further found that the strain of the invention (see example 1): (a) was able to produce siderophores; (b) produced enzymes such as leucine arylamidase, α-glucosidase and α-mannosidase; (c) was a biofilm producer; and (d) is environmental friendly
The production of siderophores as well as of the enzymes leucine arylamidase, α-glucosidase and α-mannosidase activities have beneficious impact on plant growth, development and reproduction. Siderophores are soluble Fe³⁺ binding agents which can also increase the availability and uptake of iron of the plant. Leucine arylamidase liberates amino acids from polymeric high-molecular-mass compounds providing a source of dissolved organic nitrogen that is an essential nutrient for the plant. α-glucosidase and α-mannosidase act breaking down complex carbohydrates into their monomers, providing a source of sugars to the plant. A biofilm is a community of microbial cells bounded by a polymeric matrix comprising polysaccharides associated with a surface which are reported to be important for soil quality, plant nutrition and plant protection.

Therefore, the strain of the present invention benefits the growth of the bacterized plant.

Altogether the strain of the present invention means a great advance in the field of biopesticides.

Thus, the first aspect of the present invention refers to a strain of *Microbacterium esteraromaticum* deposited at the "Colección Española de Cultivos Tipo" (CECT) under the accession number CECT9167

In the present invention the terms "B24" and "CECT9167", referring to the strain of the invention, are used interchangeably.

The strain of the invention was isolated from an agricultural soil in Almeria (South of Spain) and was deposited, according to the Budapest Treaty, at the CECT in the Universidad de Valencia C.P 46980 Catedrático Agustin Escardino N° 9 Paterna, Valencia (Spain), under the accession number CECT9167. It was deposited by the depositor Futureco Bioscience S.A., Av. Del Cadi 19-23 P.I. Sant Pere Molanta 08799 Olèrdola Barcelona, on the 13 July 2016. The strain was identified by the depositor with the reference B24, and received the accession number CECT9167. It was, in addition, declared viable.

A second aspect of the present invention refers to a bacterial culture comprising the strain as defined in the first aspect of the invention.

Due to the pesticidal effect of the strain of the present invention, it can be used as a phytosanitary product. As it is illustrated in the examples below, the strain of the first aspect of the invention can be used as a phytosanitary product according to FAO guidelines, which are the guidelines emitted by Food and Agriculture Organization of the United Nations. The isolated bacteria as defined in the first aspect of the invention may be used as ingredient of a phytosanitary composition.

Therefore, a third aspect of the present invention refers to a composition comprising an effective amount of the strain as defined in the first aspect of the invention, or the bacterial culture as defined in the second aspect of the invention, and one or more agriculturally acceptable compound(s).

A fourth aspect of the invention refers to a process for obtaining a viable cell suspension of the strain CECT9167 of *M. esteraromaticum,* of the first aspect of the invention; the process comprising: (i) inoculating the strain in a suitable culture medium, (ii) subjecting the inoculated culture medium of the step (i) to conditions suitable for growth of the strain, and (iii) optionally subjecting the medium resulting from step (ii) to a concentration step.

As indicated before, the nematicidal effect is due to the fact that the strain of the present invention releases at least certain enzymes and participates in the ISR of the plant. Therefore, a fifth aspect of the invention refers to a supernatant of the strain CECT9167 of *M. esteraromaticum* as defined in the first aspect of the invention, said supernatant being obtainable by a process comprising: (i) inoculating the strain in a suitable culture medium; (ii) subjecting the inoculated culture medium to suitable growth conditions; (iii) separating the cells from the culture medium of step (ii); (iv) collecting the supernatant; and (v) optionally subjecting the supernatant to a concentration step.

A sixth aspect of the invention refers to a kit that comprises an effective amount of the strain CECT9167 of *Microbacterium esteraromaticum* of the first aspect of the invention or the bacterial culture of the second aspect of the invention or the composition of the third aspect of the invention or the supernatant of the fifth aspect of the invention.

A seventh aspect of the present invention refers to the use of the strain CECT9167 of *Microbacterium esteraromaticum* of the first aspect of the invention or the supernatant of the fifth aspect of the invention or the kit of the sixth aspect of the invention for controlling a nematode infection in a plant.

An eighth aspect of the present invention refers to a method for controlling an infection caused by nematodes in a plant comprising applying to a part of a plant or to the substrate used for growing said plant the strain CECT9167 of *Microbacterium esteraromaticum* of the first aspect of the invention, or the bacterial culture of the second aspect of the invention, or the composition of the third aspect of the invention, or the supernatant of the fifth aspect of the invention, or the kit of the sixth aspect of the invention.

### Brief description of the drawings

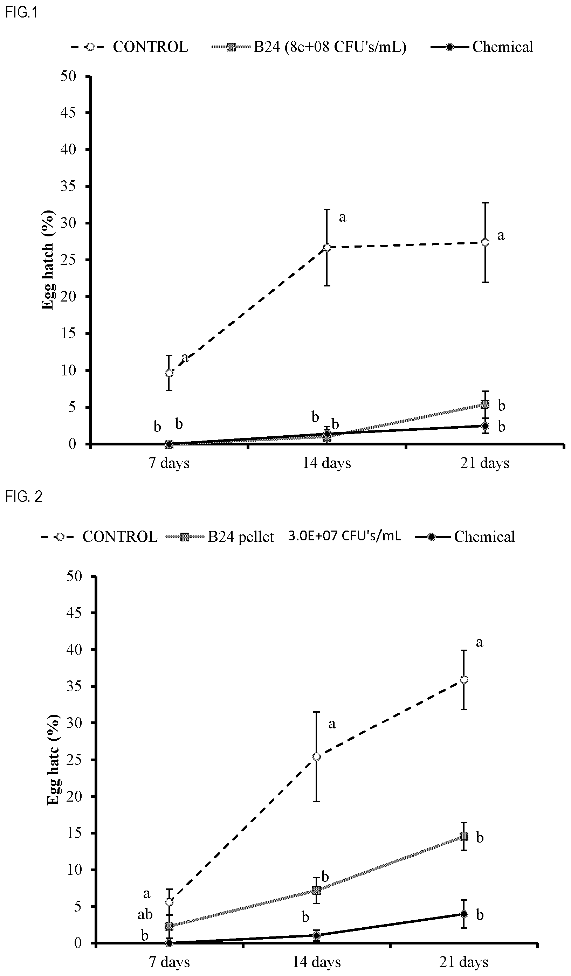

Fig.1 represents egg hatching of *M. javanica* and *M. incognita* eggs (50% of each species) after *in vitro* treatment using *M. esteraromaticum* strain B24 (at 8.0x10⁸ CFU/ml) and Nemacur^{®} ("chemical") or a negative control.
Fig. 2 represents egg hatching of *M. javanica* and *M. incognita* eggs (50% of each species) after *in vitro* treatment with *M. esteraromaticum* strain B24 (at 3x10⁷ CFU/mL), Nemacur^{®} ("chemical") or a negative control.

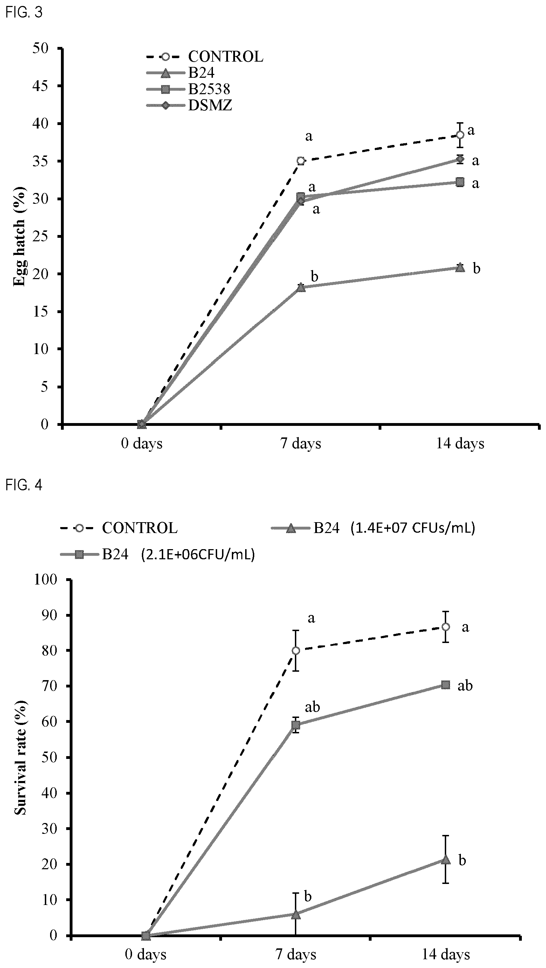

Fig. 3 shows egg hatching of *M. javanica* and *M. incognita* eggs (50% of each species) after *in vitro* treatment with *M. esteraromaticum* B24 (at 5.20x10⁷ CFU/mL), B2538 (6.2x10⁷ CFU/mL) or DSMZ (DSMZ 8609) (at 2.03x10⁷ CFU/mL) strains or a negative control.
Fig. 4 shows survival rate of *M. javanica* and *M. incognita* juveniles (J₂) (50% of each species) after *in vitro* treatment with M. *esteraromaticum* B24 strain at two concentrations (triangle, 1.4x10⁷ CFUs/mL; square, 2.1x10⁶ CFUs/mL).

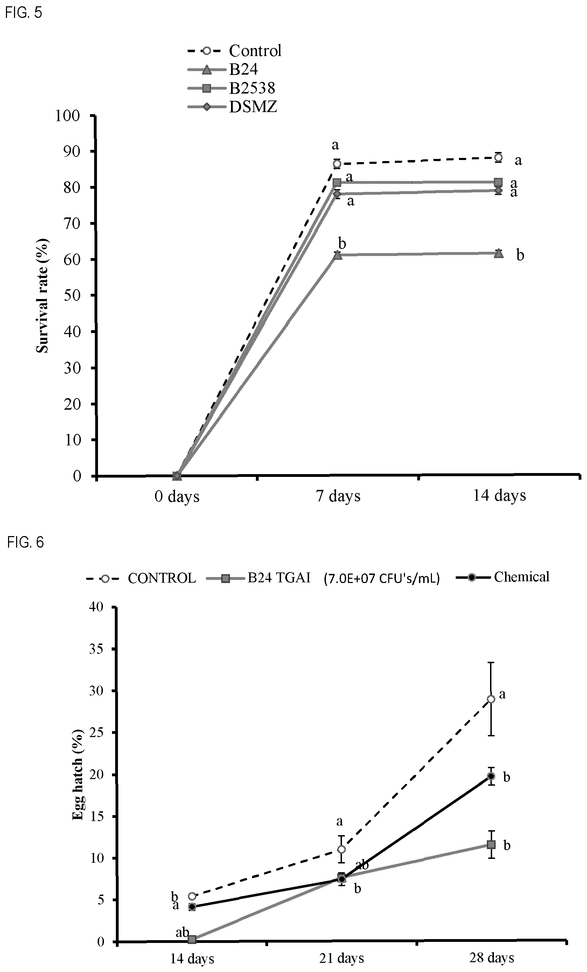

Fig.5 shows survival rate of *M. javanica* and *M. incognita* juveniles (J₂) (50% of each species) after *in vitro* treatment with *M. esteraromaticum* B24 (at 5.2x10⁷ CFU/mL), B2538 (at 6.2x10⁷ CFU/mL) or DSMZ (DSMZ 8609) (at 2.03x10⁷ CFU/mL) strains.
Fig. 6 represents a comparison of the percentage of efficacy *in vitro* on egg hatching inhibition of G. *rostochiensis* and *G. pallida* eggs of the *M. esteraromaticum* strain B24 (at 7.0x10⁷ CFU/mL) with respect to the control and a chemical product (Vydate^{®}).

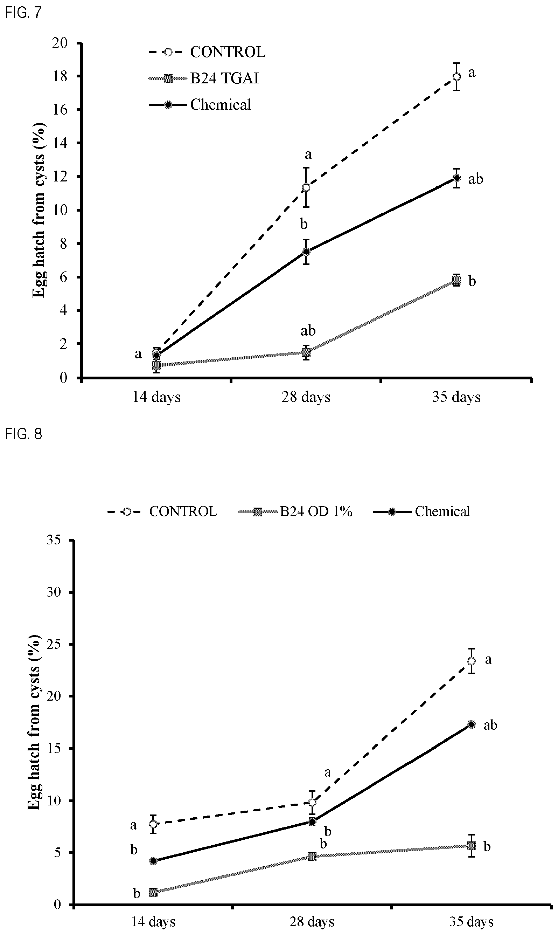

Fig. 7 represents a comparison of the percentage of efficacy *in vitro* on egg hatch from cysts of G. *rostochiensis* and *G. pallida* of the *M. esteraromaticum* strain B24 applied as TGAI (at 7.0x10⁷ CFU/mL) with respect to the control and a chemical product (Vydate^{®}).
Fig. 8 represents a comparison of the percentage of efficacy *in vitro* on egg hatch from cysts of G. *rostochiensis* and *G. pallida* of the *M. esteraromaticum* strain B24 applied as OD Formulation at 1% (2.2x10⁸ CFU/mL) with respect to the control and a chemical product (Vydate^{®}).

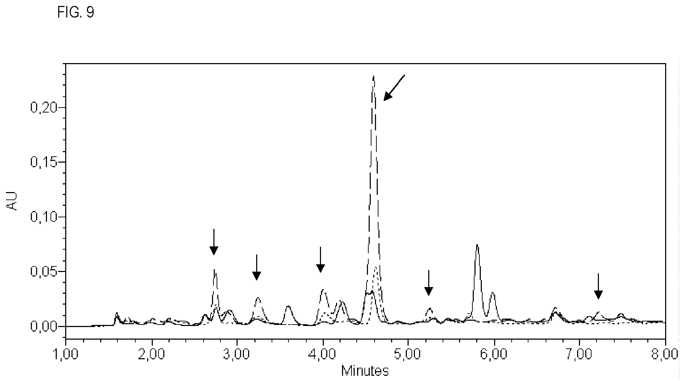

Fig. 9 represents an HPLC chromatogram at 254 nm of CS of B24-Flask (dashed line), CS of DSM 8609-Flask (dotted line) and compared with culture medium without microbial inoculation (solid line). Relevant different peaks were labelled with arrows.

In all the figures: data represented with different letter ("a", "b" or "ab") indicate that there was a statistically significant difference among them (data were subjected to analysis of variance (ANOVA) using R program; treatment means were compared using Fisher's protected least significant difference test (LSD) at P=0.05). "Control": negative control. "Egg hatch (%)": percentage of egg hatching.

### Detailed description of the invention

All terms as used herein in this application, unless otherwise stated, shall be understood in their ordinary meaning as known in the art. Other more specific definitions for certain terms as used in the present application are as set forth below and are intended to apply uniformly throughout the specification and claims unless an otherwise expressly set out definition provides a broader definition. The definitions given herein are included for the purpose of understanding and expected to be applied throughout description, claims and drawings.

The first aspect of the invention refers to an isolated strain of *Microbacterium esteraromaticum* deposited at the "Colección Española de Cultivos Tipo" (CECT) under the accession CECT9167.

By using the deposited strain as starting material, the skilled person in the art can routinely, by genetic engineering techniques such as mutagenesis or bacterial recombination techniques, obtain mutants that maintain the herein described relevant features and advantages of the strain of the invention. Such a mutant may be a genetically modified mutant obtained by random mutagenesis (i.e. using chemical or physical agents) or by site-directed mutagenesis, combinatorial mutagenesis or insertional mutagenesis. Alternatively, a mutant may be obtained by using recombinant technology, for example using transformation (for example, by electroporation, heat-shock or using divalent cation solutions, such as calcium chloride), transduction or conjugation techniques. By using recombinant technology a plasmid can be included in the bacterial strain, said plasmid can comprise antibiotic resistant genes or genes that serve for the selection of the mutant. Examples of genetic engineering techniques can be found in Sambrook, J. and Russell, D.W. "Molecular Cloning: A Laboratory Manual", Chapter 13, "Mutagenesis", Cold Spring Harbor, 3rd Ed, 2001.

However, the mutants and their uses do not form part of the invention.

In order to determine whether the mutant maintains the broad anti-nematode profile, several well-known protocols can be followed. These methods are commonly based on the analysis of the growth capacity of the pathogen in contact with the strain, or the assessment of the severity of the disease caused by the pathogen infection after the exposition to the strain. The protocols that are included in the examples herein for the determination of the activity are illustrative and non-limitative examples. Briefly, they are based on *in vitro* nematode egg-hatching and nematode survival; and *in vivo* nematode reproduction analysis.

In a particular embodiment of the first aspect of the invention, optionally in combination with any of the embodiments provided above or below, the nematicidal effect is against at least a nematode of the genus *Meloidogyne, Globodera and*/*or Pratylenchus.* In another embodiment, optionally in combination with any of the embodiments provided above or below, the nematicidal effect is against at least a nematode of the genus *Meloidogyne* and *Globodera.* In another embodiment, optionally in combination with any of the embodiments provided above or below, the nematicidal effect is against one or more of *Meloidogyne incognita, Meloidogyne javanica, Globodera rostochiensis* and *Globodera pallida.* In another embodiment, the strain as defined in the first aspect of the invention has a nematicidal effect against *Meloidogyne incognita, Meloidogyne javanica, Globodera rostochiensis* and *Globodera pallida.* In another embodiment, optionally in combination with any of the embodiments provided above or below, the nematicidal effect is against one or more of *Pratylenchus penetrans, Pratylenchus fallax, Pratylenchus coffeae, Pratylenchus loosi,* and *Pratylenchus vulnus.*

In another embodiment of the first aspect of the invention, optionally in combination with any of the embodiments provided above or below, the strain as defined in the first aspect of the invention has a nematicidal effect against *Meloidogyne incognita, Meloidogyne javanica, Globodera rostochiensis* and *Globodera pallida.*

A second aspect of the invention refers to a bacterial culture comprising the strain as defined in the first aspect of the invention.

In an embodiment of the second aspect of the invention, optionally in combination with any of the embodiments provided above or below, the bacterial culture comprises a viable strain as defined in the first aspect of the invention.

In an embodiment of the second aspect of the invention, optionally in combination with any of the embodiments provided above or below, the bacterial culture is an inoculation product.

By "inoculation product" it is understood a product obtained after inoculating the strain in a suitable culture medium, subjecting the inoculated culture medium to suitable growth conditions.

In another embodiment of the second aspect of the invention, optionally in combination with any of the embodiments provided above or below, the bacterial culture comprises the strain of the invention inactivated.

The term "inactivated" means that the micro-organism is not able to form colonies. In one embodiment, the inactivated micro-organisms have the cell membrane intact or broken.

In another embodiment of the second aspect of the invention, optionally in combination with any of the embodiments provided above or below, the inoculation product comprises the strain of the invention inactivated.

By "inoculation product comprising the strain of the invention inactivated" refers to a product obtained after inoculating the strain in a suitable culture medium, subjecting the inoculated culture medium to suitable growth conditions, and then inactivating the strain.

With a view to practical use in pest control, pesticide agents are usually formulated into compositions also including agriculturally acceptable compounds. Therefore, a third aspect of the present invention refers to a composition comprising an effective amount of the strain as defined in the first aspect of the invention, or the bacterial culture as defined in the second aspect of the invention, and one or more agriculturally acceptable compounds.

The term "effective amount" as used herein, means an amount of the strain M. *esteraromaticum* CECT9167 as defined in the first aspect of the invention, high enough to provide the desired benefit, either the treatment or prevention of the plant disease, but low enough to avoid serious side-effects. The particular dose of compound administered according to this invention will of course be determined by the particular circumstances surrounding the case, including the compound administered, the route of administration, the particular condition being treated, and the similar considerations.

In an embodiment of the third aspect of the invention, optionally in combination with any of the embodiments provided above or below, the strain is present at a concentration from 1.0x10⁵ CFUs/mL to 1.0x10¹² CFUs/mL. In another embodiment of the third aspect of the invention, the strain is present at a concentration of 1.0x10⁵ CFUs/mL, 1.0x10⁶ CFUs/mL, 1.0x10⁷ CFUs/mL, 1.0x10⁸ CFUs/mL, 1.0x10⁹ CFUs/mL, 1.0x10¹⁰CFUs/mL, 1.0x10¹¹ CFUs/mL or 1.0x10¹²CFUs/mL.

For the purposes of the invention, any ranges given include both the lower and the upper end-points of the range.

The amount indicated as CFU/mL relates to the colony forming units (CFU or CFUs) of the strain of the invention per milliliter or gram of the composition.

In another embodiment of the third aspect of the invention, optionally in combination with any of the embodiments provided above or below, the composition is a phytosanitary composition.

In another embodiment of the third aspect of the invention, optionally in combination with any of the embodiments provided above or below, the composition comprises at least one additional pesticide.

In another embodiment of the third aspect of the invention, optionally in combination with any of the embodiments provided above or below, the additional pesticide is selected from the group consisting of another bacterial strain with pesticide properties, a fungicide, a bactericide, an herbicide, an insecticide or a chemical nematicide. Said additional pesticide does not have to adversely affect the activity/viability of the strain of the invention included in the composition.

In the present invention, the term "pesticide" is understood by its usual meaning in the field of agronomy as a product intended to kill, repel, regulate or disrupt the growth of living organisms that are considered pests. Clearly, due to the nature of the strain CECT9167 of M. *esteraromaticum,* herein it is understood that "pesticide" is a biological or ecological (organic) pesticide, also called biopesticide. In the scope of the present invention, the term "pesticide" would have the same meaning as the term "phytosanitary".

In an embodiment of the third aspect of the invention, optionally in combination with any of the embodiments provided above or below, the composition can be in the form of a solution, a pellet, a suspension, a lyophilized compositions or other dried compositions (for example freeze dried composition). The lyophilized or dried composition can be reconstituted with a liquid carrier prior to its use or directly used. The composition may be prepared according to various formulations suitable for phytosanitary uses, for example, chosen from the group consisting of formulations of the following type: liquid intended for use without dilution (AL), powder intended for use without dilution (AP), encapsulated granule (CG), contact liquid or gel (CL), contact powder (CP), powdering powder (DP), emulsifiable concentrate (EC), emulsifiable granule (EG), oil type emulsion (EO), water type emulsion (EW), fine granule (FG), macrogranules (GG), emulsifiable gel (GL), powder for spraying (GP), granules (GR), grease (GS), water-soluble gel (GW), microemulsion (ME), microgranules (MG), water-dilutable concentrated suspension (OF), oil dispersion (OD), water-miscible suspension (OL), powder for dispersion in oil (OP), concentrated in gel or paste form (PC), sticks (for agri-pharmaceutical use) (PR), concentrated suspension (SC), suspoemulsion (SE), water-soluble granules (DG), soluble concentrate (SL), film-forming oil (SO), water-soluble powder (SP), water-soluble tablets (ST), tablets (TB), water-dispersible granules (WG), wettable powder (WP), water-dispersible tablets (WT) (the code consisting of two capital letters corresponding to the international codes for phytosanitary formulations).

In an embodiment of the third aspect of the invention, optionally in combination with any of the embodiments provided above or below, the composition is an OD composition.

In an embodiment of the third aspect of the invention, optionally in combination with any of the embodiments provided above or below, the composition is an OD formulation and comprises a concentration of the strain as defined in the first aspect of the invention in the range from 1.0x10⁵ to 1.0x 10¹² CFU/mL (1.0x10⁵, 1.0x10⁶, 1.0x10⁷, 1.0x10⁸, 1.0x10⁹, 1.0x10¹⁰, 1.0x10¹¹ or 1.0x10¹² CFU/mL).

In an embodiment of the third aspect of the invention, optionally in combination with any of the embodiments provided above or below, the composition is an OD formulation and comprises a concentration of the strain as defined in the first aspect of the invention of 10¹⁰ CFU/mL.

In an embodiment of the third aspect of the invention, optionally in combination with any of the embodiments provided above or below, the composition is an OD formulation and comprises a concentration of the strain as defined in the first aspect of the invention in the range from 1.0x10⁵ to 1.0x10¹² CFU/mL and also comprises a vegetable oil, an organic ester, silica, and a high molecular weight copolymer.

In an embodiment of the third aspect of the invention, optionally in combination with any of the embodiments provided above or below, the composition comprises an oily ingredient. In another embodiment, the oily ingredient is a vegetable oil. In another embodiment, the vegetable oil is soy oil.

In an embodiment of the third aspect of the invention, optionally in combination with any of the embodiments provided above or below, the composition comprises soy oil, C18 ethoxilated fatty acid, silica and polyacrylate crosspolymer.

In another embodiment of the third aspect of the invention, optionally in combination with any of the embodiments provided above or below, the composition is an OD composition which comprises soy oil, C18 ethoxilated fatty acid, silica, polyacrylate crosspolymer and comprises a concentration of the strain as defined in the first aspect of the invention in the range from 1.0x10⁵ to 1.0x10¹² CFU/mL.

In another embodiment of the third aspect of the invention, optionally in combination with any of the embodiments provided above or below, the composition is an OD composition which consists of 70% of soy oil, 20% of C18 ethoxylated fatty acid, 1% silica, polyacrylate crosspolymer and 8% of the bacterial strain of the present invention; in another embodiment, the final concentration of the bacteria is 1x10¹⁰ CFU/mL.

"Agriculturally acceptable compounds" refers to those compounds and/or materials, which are suitable for use in agriculture. In general, said compounds should be non-toxic to humans and preferably should be environment-friendly.

In a particular embodiment of the third aspect of the invention, optionally in combination with any of the embodiments provided above or below, the compositions of the invention may contain compounds for improving the adhesion of the strains in the plants to be treated, as well as phytostrengthener compounds, nutrients, wetting agents, stabilizers, osmotic protectors, antioxidants, sunscreens, buffering compounds or combinations thereof.

Examples of adhesion products are gelatin, starch, pectins, alginates and various types of gums such as xanthan. Many of these compounds are also wetting agents. In the case of sunscreens, Congo red, calcium carbonate and wax emulsions can be used. The phytostrengtheners are compounds that can facilitate make crops develop robustness or tolerance towards pathogens or adverse environmental conditions, for example, jasmonic acid analogues and some plant defense stimulants such as harpins, chitosans, and laminarins. Additionally, examples of osmotic protectors are trehalose, betaines and amino acids. Finally, ascorbic acid and glutathione are included among antioxidants.

The compositions of the third aspect of the invention can be prepared by routine protocols, such as by mixing the different ingredients.

A fourth aspect of the invention refers to a process for obtaining a viable cell suspension derived from the strain CECT9167 of *M. esteraromaticum* of the first aspect of the invention, the process comprising: (i) inoculating the strain in a culture medium, (ii) subjecting the inoculated culture medium of the step (i) to conditions suitable for growth of the strain, and (iii) optionally subjecting the medium resulting from step (ii) to a concentration step.

The term "derived from the strain CECT9167" means that the suspension is obtained from the strain as defined in the first aspect of the present invention.

The strain of the invention may be inoculated in the culture medium at a final concentration comprised from 3 to 7% v/v, for example from 5 to 7% v/v, for example of 5% v/v. Preferably the inoculated culture is in an exponential growth phase. Suitable culture media for the growth of the strain of the invention are synthetic media, such as LB (lysogenic broth) and PM (saline production medium), or media of plant origin such as molasses (e.g. from sugar cane, beets and others). Suitable conditions for strain's growth are temperatures comprised from 6 to 42 °C, pH comprised 4 to 10 (for example 7 to 10), and oxygen concentration comprised from 10 to 100%. In a particular embodiment, the conditions for the growth of the strain are temperatures of 28-30 °C, pH of 7-8, and oxygen concentration of 50%. During the growth of the strain of the invention stirring can be used.

In another embodiment of the fourth aspect of the invention, optionally in combination with any of the embodiments provided above or below, cells are separated from the medium to obtain a concentrated suspension. Suitable separation techniques include centrifugation or filtration of the culture. Carrying out the centrifugation of the culture, for example, at a minimum of 5000 rpm, cells are obtained in the pellet, which are resuspended in part of the culture medium or in a suitable buffered medium such that the strain concentration is approximately about 1x10⁵ - 1x10¹² CFU/mL (10⁵ CFU /mL, 10⁶ CFU /mL, 10⁷ CFU /mL, 10⁸ CFU /mL, 10⁹ CFU /mL, 10¹⁰ CFU /mL, 10¹¹ CFU /mL or 10¹² CFU /ml).

Once the suspension is obtained, it may be subjected to a dehydration step. Dehydration can be carried out through a lyophilisation process. Alternatively, the suspension can be dehydrated by fluidised bed drying. Another option is to dehydrate the suspension by spray drying or drying in an oven under vacuum. In this regard, another advantageous feature of the strain of the invention is that it exhibits high resistance to dehydrating processes, which are routine in obtaining microorganisms on an industrial scale. In order to improve cell viability, an inert osmotic protector ingredient can be added to the suspension before carrying out the dehydration process.

In another particular embodiment of the fourth aspect of the invention, optionally in combination with any of the embodiments provided above or below, the process comprises re-suspending the cells resulting from the separation step in a suitable buffer to yield a cell concentrated suspension.

The cells obtained with the fourth aspect of the invention may then be used directly, resuspended to a desired density, subjected to dehydration or disrupted to obtain a cell free extract.

A fifth aspect of the present invention refers to a supernatant derived from the strain CECT9167 of *M. esteraromaticum* as defined in the first aspect of the invention, said supernatant being obtainable by a process comprising: (i) inoculating the strain in a suitable culture medium; (ii) subjecting the inoculated culture medium to suitable growth conditions; (iii) separating the cells from the culture medium of step (ii); (iv) collecting the supernatant; and (v) optionally subjecting the supernatant to a concentration step.

In a particular embodiment of the fifth aspect of the invention, optionally in combination with any of the embodiments provided above or below, the supernatant is from the strain CECT9167 of *M. esteraromaticum* as defined in the first aspect of the invention

In a particular embodiment of the fifth aspect of the invention, optionally in combination with any of the embodiments provided above or below, the supernatant is produced by the strain CECT9167 of *M. esteraromaticum* as defined in the first aspect of the invention, and comprises N-acetyl-β-glucosaminidase, leucine arylamidase, α-glucosidase and α-mannosidase, for example, when the culture medium is liquid medium, for example is tryptic soy broth (TSB).

Another aspect of the present invention provides a cell extract derived from the strain CECT9167 of *M. esteraromaticum* or a mutant thereof as defined in the first aspect of the invention, said cell extract being obtainable by a process comprising: (i) inoculating the strain in a suitable culture medium; (ii) subjecting the inoculated culture medium to suitable growth conditions; (iii) separating the cells from the culture medium of step (ii); (iv) collecting the cells and (v) disrupting the cells, (vi) separating the cell extract from cell debris, (vii) collecting the cell extract, and (viii) optionally subjecting the cell extract to a concentration process.

Suitable disrupting means are known by the skilled person and may include physical disruption, for example freeze-thaw or French Press, or chemical disruption, for example by addition of lysozyme. Suitable separation means have been described above. The cell free extract may also be obtained from a cell suspension as defined above, preferably containing metabolite-containing supernatant.

The supernatant of the fifth aspect of the invention or the cell extract obtained by the methods described above, could be used and/or included in an appropriated composition directly or subjected to a concentration step to reach a more suitable composition. Thus, in an embodiment of said aspects of the invention the step of concentration of the supernatant or, alternatively, of the cell extract can be performed by dehydration, filtration, ultra-filtration, centrifugation, ultra-centrifugation, precipitation or chromatography.

A sixth aspect of the present invention relates to a kit that comprises an effective amount of the strain, as defined in the first aspect of the invention, or the bacterial culture of the second aspect of the invention or the composition of the third aspect of the invention or the supernatant of the fifth aspect of the invention.

All the embodiments provided above for the strain of the first aspect of the invention, the bacterial culture of the second aspect of the invention and the composition of the third aspect of the invention are also embodiments of the kit of the sixth aspect of the invention.

In an embodiment of the sixth aspect of the invention, optionally in combination with any of the embodiments provided above or below, the kit may comprise a weekly, monthly, or other periodic dose of the strain of the first aspect of the invention. As an illustrative example, a kit comprising a weekly dose may comprise seven discrete compositions comprising the strain (seven daily doses). As another example, a kit comprising a monthly dose may comprise thirty compositions comprising the strain of the first aspect of the invention.

In case the strain of the first aspect of the invention is lyophilized, the kit of the sixth aspect of the invention can contain a resuspension agent, such as water.

In another embodiment of the sixth aspect of the invention, optionally in combination with any of the embodiments provided above or below, the kit comprises means to facilitate dosing compliance. For example, the kits may be particularly advantageous for the purpose of ensuring that the person who used it is performing the correct administration of the effective amount of the strain of the invention to the plant on the appropriately prescribed schedule. Blister cards or other containing devices appropriately configured may be particularly suitable for clearly illustrating sequence or timing of administration of the various components. The kit may be obtained as one card, or cases of four, six, seven (e.g., a weekly supply), or eight cards co-packaged together. Additionally, monthly or other types of kits may be obtained.

The kit that comprises the isolated strain, of the first aspect of the invention can comprise any means that allows the correct culture of the strain of the invention, such as culture medium, supplements or antibiotics, as well as instructions for the correct preparation and/or application to the plant.

A seventh aspect of the invention refers to the use of the strain CECT9167 of *Microbacterium esteraromaticum* of the first aspect of the invention, or the supernatant of the fifth aspect of the invention, or the kit of the sixth aspect of the invention for controlling a nematode infection in a plant.

A eighth aspect of the present invention refers to a method for controlling an infection caused by nematodes in a plant comprising applying to a part of a plant or to the substrate used for growing said plant the strain CECT9167 of *Microbacterium esteraromaticum* of the first aspect of the invention or the supernatant of the fifth aspect of the invention, or the kit of the sixth aspect of the invention.

The strain of the seventh and eighth aspects of the invention can be used as a bacterial culture or as a composition.

All the embodiments provided above for the strain of the first aspect of the invention, the bacterial culture of the second aspect of the invention and the composition of the third aspect of the invention are also embodiments of the seventh and eighth aspects of the invention.

In an embodiment of the eighth aspect of the invention, optionally in combination with any of the embodiments provided above or below, the strain CECT9167 of M. *esteraromaticum,* as defined in the first aspect of the invention is administered to the plant at a dosage regime of 10⁶ - 10⁸ CFU/g or 10⁶ - 10⁸ CFU/mL per day once during several weeks, for example 10⁶ - 10⁸ CFU/g or 10⁶ - 10⁸ CFU/mL 1-6 times in several weeks, or for example 10⁶ - 10⁸ CFU/g or CFU/mL 1-4 times per treatment.

The dose may be adapted according to the composition of the third aspect of the invention and the formulation of the composition which is used and also according to the weather conditions, any resistance phenomena or other natural factors, the nature of the treatment or the degree of infestation, and according to the plants or sites to be treated.

In an embodiment of the eighth aspect of the invention, optionally in combination with any of the embodiments provided above or below, the strain, the bacterial culture or the composition is applied to plant nursery trays, during seed treating, seed dressing, seed disinfection, seedling root dipping treatment, planting pit treatment, plant foot treatment, planting row treatment, surface spraying, soil incorporation, drip irrigation, or by application to a water culture medium in hydroponic culture.

The term "part of a plant" includes any segment of the plant, such as the root, stem, leaves and seeds.

In an embodiment of the eighth aspect of the invention, optionally in combination with any of the embodiments provided above or below, the part of the plant treated is the root system (root).

In an embodiment of the eighth aspect of the invention, optionally in combination with any of the embodiments provided above or below, the part of the plant treated is the seed. This treatment can be achieved by ordinary methods; for example, a method wherein seeds are dipped in the composition of the third aspect of the invention, coating the seeds.

The term "seed" includes what is called seeds, and also plant bodies for vegetative propagation such as bulbs, tubers and seed potato.

The treatment of the plant, i. e. root and/or the seeds, allows the nematicidal action of the strain of the invention against root-nematodes.

The term "plant" comprises all plant species cultivated by humans, in particular those intended for food or for animal feed, such as cereals, fodder, vegetable, fruit crops, vines, and/or for the supply of wood for all purposes (such as heating, housing construction furniture, and/or ornamentation). Example of plants include cereals (for example, rice, barley, wheat, rye, oat and corn), beans (for example, soybean, azuki bean, broad bean, peas and peanuts), fruit trees/fruits (for example, apples, pears, citruses, grapes, peaches, apricots, cherries, olive, nuts, almonds, bananas, berries and strawberries), vegetables (for example, tomato, cabbage, spinach, broccoli, lettuce, onion, garlic, leek and pepper), root crops (for example, potato, carrot, sweet potato, radish, lotus root and turnip), industrial crops (for example, cotton, hemp, paper mulberry, mitsumata, rape, beet, hop, sugarcane, sugar beet, rubber, coffee, tobacco, tea), pepos (for example, pumpkin, cucumber, watermelon, melon), pasture plants (for example, orchardgrass, sorghum, Thimothy-grass, clover, alfalfa), lawn grasses (for example, mascarene grass, bentgrass), crops for flavorings (for example, lavender, rosemary, thyme, parsley, basilica, mint, coriander, pepper, ginger), and flower plants (for example, chrysanthemum, rose, orchids).

In an embodiment of the seventh or eighth aspect of the invention, optionally in combination with any of the embodiments provided above or below, the plant is tomato, cucumber or potato plant.

The term "substrate" comprises any support for cultivating a plant, and the material therefor is not particularly limited, as far as the plant can grow therein; for example, nursery mats, water, sand, soil, vermiculite, cotton, paper, diatomaceous earth, agar, gel substances, polymeric substances, rock wool, glass wool, wood chips, barks and pumice.

In an embodiment of the eighth aspect of the invention, optionally in combination with any of the embodiments provided above or below, the method of application is performed in the vicinity of the plant or a nursery bed for raising seedlings.

In an embodiment of the eighth aspect of the invention, optionally in combination with any of the embodiments provided above or below, the method of application is performed directly in the plant. In an embodiment, the method of application is performed on the root system and/or the seeds.

The examples below demonstrate the use of a strain of *M. esteraromaticum* as a bio-pesticide, in the treatment of nematode infection.

In an embodiment of the seventh and eighth aspects of the invention, optionally in combination with any of the embodiments provided above or below, the use of the seventh aspect and the method of the eighth aspect are for controlling the colonization of pest nematodes or treating infested plants. In particular, the invention relates to uses for controlling those plant pests. The term "treating" therefore is referred to infested plants. The term "control" comprises preventing infestations of the plants by said pests, repelling or eliminating said pests.

In an embodiment of the seventh and eighth ghthaspects of the invention, optionally in combination with any of the embodiments provided above or below, the use of the seventh aspect and the method of the eighth aspect are for treating a plant infected by pest nematodes.

In an embodiment of the seventh and eighth aspects of the invention, optionally in combination with any of the embodiments provided above or below, the nematode is a root-knot nematode or a cyst nematode.

In an embodiment of the seventh and eighth aspects of the invention, optionally in combination with any of the embodiments provided above or below, the nematode is of the genus *Meloidogyne,* such as *Meloidogyne incognita* (southern root-knot nematode), *Meloidogyne javanica* (Javanese root-knot nematode), *Meloidogyne hapla* (northern root-knot nematode), and *Meloidogyne arenaria* (peanut root-knot nematode); nematodes of the genus *Globodera,* such as *Globodera rostochiensis* (golden nematode) and *Globodera pallida* (potato cyst nematode); nematodes of the genus *Ditylelenchus,* such as *Ditylelenchus destructor* (potato rot nematode) and *Ditylelenchus dipsaci* (bulb and stem nematode); nematodes of the genus *Pratylenchus,* such as *Pratylenchus penetrans* (cobb root-lesion nematode), *Pratylenchus fallax* (chrysanthemum root-lesion nematode), *Pratylenchus coffeae* (coffee root-lesion nematode), *Pratylenchus loosi* (tea root-lesion nematode), and *Pratylenchus vulnus* (walnut root-lesion nematode); nematodes of the genus *Heterodera,* such as *Heterodera glycines* (soybean cyst nematode) and *Heterodera shachtoii*) (sugar beet cyst nematode); nematodes of the genus *Aphelenchoides,* such as *Aphelenchoides besseyi* (rice white-tip nematode), *Aphelenchoides ritzemabosi* (chrysanthemum foliar nematode), and *Aphelenchoides fragarieae* (strawberry nematode); nematodes of the genus *Aphelenchus,* such as *Aphelenchus avenae* (mycophagous nematode); nematodes of the genus *Radopholus,* such as *Radopholus similis* (burrowing nematode); nematodes of the genus *Tylenchulus,* such as *Tylenchulus semipenetrans* (citrus nematode); nematodes of the genus *Rotylenchulus,* such as *Rotylenchulus reniformis* (reniform nematode); or nematodes that occur in trees, such as *Bursaphelenchus xylophilus* (pine wood nematode).

In an embodiment of the eighth and ninth aspects of the invention, optionally in combination with any of the embodiments provided above or below, the nematode is selected of the genus *Meloidogyne;* or alternatively the nematode is of the genus *Globodera.*

In an embodiment of the seventh and eighth aspects of the invention, optionally in combination with any of the embodiments provided above or below, the nematode is selected form the group consisting of *Meloidogyne incognita, Meloidogyne javanica, Globodera rostochiensis* and *Globodera pallida;* or, alternatively, the nematode is *Meloidogyne incognita;* or, alternatively, the nematode is *Meloidogyne javanica;* or, alternatively, the nematode is *Globodera rostochiensis;* or, alternatively, the nematode is *Globodera pallida.*

In the present invention the term "infection" comprises asymptomatic infection or symptomatic infection of the plant caused by a nematode.

In an embodiment of the seventh and eighth aspects of the invention, optionally in combination with any of the embodiments provided above or below, the control can be performed in any plant.

Throughout the description and claims the word "comprise" and variations of the word, are not intended to exclude other technical features, additives, components, or steps. Furthermore, the word "comprise" encompasses the case of "consisting of'. Additional advantages of the invention will become apparent to those skilled in the art upon examination of the description or may be learned by practice of the invention. The following examples and drawings are provided by way of illustration, and they are not intended to be limiting of the present invention. Reference signs related to drawings and placed in parentheses in a claim, are solely for attempting to increase the intelligibility of the claim, and shall not be construed as limiting the scope of the claim.

### Examples:

### Example 1: Characterization of the M. esteraromaticum strain B24

### 1.1 Species identification:

### Materials and methods

The strain B24 of *M. esteraromaticum* was isolated in an agricultural soil under organic farming regime from Almeria (South of Spain). Identification of the bacterial isolate was achieved using 16S rRNA gene sequence analysis.

The 16S rRNA gene sequencing was performed as described next. A PCR reaction mix, which consisted of 10 µL Phire Green Hot Start II Master Mix (Thermo Fisher Scientific), 0.8 µL primer 8f 10 µM (SEQ ID NO:1), 0.8 µL primer 1492r 10 µM (SEQ ID NO:2), 2 µL template (a single colony picked from a nutritive agar plate, resuspended in 50 µL sterile distilled water, boiled for 10 min at 98 °C, and chilled to 4 °C) and 6.4 µL nanopure water, was amplified according to the following program (direct PCR): 98°C 5 min; 35 cycles of 98°C 5 s, 58.5°C 5 s and 72°C 20 s, and 72°C 20 s. The PCR product was purified using the EZNA Cycle Pure Kit (Omega Bio-Tek) according to manufacturer's instructions. The purified PCR product was sequenced by an external sequencing service (Secugen) using the primer 1492r (SEQ ID NO: 2) and the Sanger method with BigDye^{®} Terminator v3.1 (Applied Biosystems) according to manufacturer's instructions. The sequencing revealed a sequence (SEQ ID NO: 3) which was 100% identical to that of several *M. esteraromaticum* strains (NCBI accession numbers MG705679.1, JF496416.1, JF496262.1, AB355700.1, AB099658.1, AB099656.1; database accession date 10/1/2018).

### 1.2 Characterization of the enzymatic activities

### Materials and methods

The enzymatic activities were determined using API^{®}ZYM (bioMérieux, Madrid, Spain), following the manufacturer's instructions. The activities of the following enzymes were tested (in braquets their corresponding substrate): Alkaline phosphatase (2-naphthyl phosphate), Esterase C 4 (2-naphthyl butyrate), Esterase Lipase C 8 (2-naphthyl caprylate), Lipase C 14 (2-naphthyl myristate), Leucine arylamidase (L-leucyl-2-naphthylamide), Valine arylamidase (L-valyl-2-naphtylamide), Cystine arylamidase (L-cystyl-2-naphtylamide), Trypsin (N-benzoyl-DL-arginine-2-naphtylamide), α-chymotrypsin (N-glutaryl-phenylalanine-2-naphtylamide), Acid phosphatase (2-naphtyl phosphate), Naphtol-AS-BI-phosphohydrolase (Naphtol-AS-BI-phosphate), α-galactosidase (6-Br-2-naphthyl-αD-glucopyranoside), β galactosidase (2-naphtyl-βD-glucopyranoside), β-glucuronidase (Naphthol-AS-BI-βD-glucuronide), α-glucosidase (2-naphtyl-αD-glucopyranoside), β-glucosidase (6-Br-2-naphthyl-βD-glucopyranoside), N-acetyl-β-glucosaminidase (1-naphthyl-N-acetyl-βD-glucosaminide), α-mannosidase (6-Br-2-naphtyl-aD-mannopyranosido) and α-fucosidase (2-naphtyl-αL-fucopyranoside).

### Results:

B24 produced leucine arylamidase, α-glucosidase and α-mannosidase. There were 13 negatives enzymatic reactions, therefore, B24 did not produce the others tested enzymes in the assay conditions.

### 1. 3: Siderophore production

### Materials and methods

Production of ferric ion chelates was detected using Chrome Azurol S dye (CAS) methodology (Schwyn B and Neilands J B "Universal chemical assay for the detection and determination of siderophores" 1987 Anal Biochem 160(1): 47-56) with some modifications. CAS solution was made by dissolving 60.5 mg CAS powder (Sigma-Aldrich) in 50 ml distilled water and 10 ml of Fe(III) solution (1 mM FeCl₃•6H₂O, 10Mm HCl) were added. Under stirring this solution was slowly mixed with 72.9 mg hexadecyltrimethylammonium bromide (HDTMA, Sigma-Aldrich), dissolved in 40 ml water and the resultant dark blue solution was autoclaved to sterilize and stored in the dark.

A CAS assay in liquid media was performed performing the following method: 900 µl of overnight bacterial cultures (grown in Luria-Bertani (LB) medium at 28°C and 200 rpm) were mixed with 100µl of the CAS assay solution in a glass tube. Mixtures were allowed to incubate for 30 min at room temperature and compared to an uninoculated media control.

A CAS agar assay was performed performing the following method: overnight bacterial cultures were grown at 28°C for 24h on King B plates (20 g/L casein peptone, 1.5 g/L K₂HPO₄, 1.5 g/L MgSO₄•7H₂O, 10 ml glycerol, and 15 g/L agar) containing 1/10 of the CAS solution.

### Results:

Following CAS assay in agar and liquid media, it was determined that B24 was able to produce siderophores. Both assays showed the change of dark blue to orange color after incubation with the bacteria strain B24 (in the CAS agar assay an orange halo formed around microbial colonies was indicative of siderophores excretion), while no change was observed after incubation with other bacteria that did not produced siderophores.

### 1.4: Biofilm formation

### Materials and methods

The strains used in this study consisted of two *M. esteraromaticum* isolates from the Futureco Bioscience collection: the strain of the invention (B24) and the strain B2538; and a reference strain, the *M. esteraromaticum* strain DSM 8609 that was purchased form the *Deutsche Sammlung von Mikroorganismen und Zellkulturen GmbH* (DSMZ) collection.

To determine biofilm formation, overnight cultures of each isolate were grown in LB at 28°C on a rotary shaker. Next day, cultures were diluted into fresh LB to give an OD₆₀₀ of 0.1. Sterile untreated 96-well microtiter plates were inoculated with 200 µL of the bacterial suspensions and incubated for 48h at 28°C. Prior to biofilm quantification, cell growth in each well was estimated by measuring the optical density at 620 nm (OD₆₂₀) using a plate reader (Multilabel Plater Reader HALO LED96, Dynamica). Quantification of the biofilm biomass was performed by crystal violet (CV) staining. Briefly, wells containing adhered cells were washed three times with water, fixed at 60°C for 1 hour and stained during 15 minutes with 200 µL of 0.1% CV. The stained biofilms were rinsed with distilled water, allowed to dry for 30 min at 37°C and then extracted with 200 µL of 95% ethanol. The amount of biofilm was quantified by measuring the OD₆₂₀ of dissolved CV using a plate reader. Biofilm formation (OD₆₂₀ of CV) was normalized by cell growth (OD₆₂₀) and reported as the relative biofilm formation (OD₆₂₀ of CV/OD₆₂₀ of the culture).

### Results:

Biofilm staining with CV at 0.1% revealed high intensity of dying in B24; however, B2538 and DSM 8609 showed very low intensity, both similar to the negative control (medium without the microorganism). The relative biofilm formation was the following: B24, 0.36; B2538, 0.05; and DSM 8609, 0.00. Therefore, B24 had higher biofilm formation than the other tested bacterial strains.

### 1.5: pH conditions of growth

### Materials and methods

pH range of growth at 28°C of strain B24 was determined in Nutritive Agar (NA) (Scharlab - UNE-EN 12780, EN ISO 16266) plates at different pH. For the experiment, B24 was first grown overnight in LB (5g/L Yeast Extract; 10g/L Tryptone; 10g/L NaCl). After that, B24 was streaked on NA plates at different pHs: 4, 5, 6, 7, 8, 9 and 10. The plates were read after 24h and 4 days of incubation at 28°C and the bacterial cell growth was evaluated visually.

### Results:

The *M. esteraromaticum* strain B24 grew in pHs: 5, 6, 7, 8, 9 and 10.

### 1.6: Supernatant of B24 strain

### Materials and methods

### Culture conditions:

*M. esteraromaticum* strain B24 was grown in liquid culture medium TSB in 14L bioreactor at 30°C, 200-450 rpm for 18 hours. For comparison purposes, the *M. esteraromaticum* collection strain DSM 8609 was used. B24 and DSM 8609 strains were grown in 125ml of half-strength TSB medium in 250ml-flask. Culture conditions were at 28°C 200rpm for 2 days. After incubation, cell-free culture supernatants (CS) were obtained by centrifugation and filtration through a 0.22 µm pore size filter. To confirm that the supernatants were free of bacterial cells, aliquots of 100 µl were platted in nutritive agar (AN) and absence of growth was confirmed after incubation at 26°C for 2 days.

### In vitro nematicidal activity against root knot nematode Meloidogyne:

The *in vitro* nematicidal activity of CS samples on nematode eggs was evaluated in hatching chambers (NunclonTM Surface multiwall plates, Nunc, Denmark) in which 2 g of previously sterilized sand was added. The substrate was then inoculated with an aqueous suspension containing 550 eggs per well (mixture of 50% *M. javanica* and 50% *M*. *incognita,* origin Viladecans, Spain). The hatching chamber was incubated at 26°C for 2 weeks and readings were done at 7 and 14 days after applying CS sample to determine the hatching percentage. Four repetitions of each treatment and a control with sterile distilled water (to establish a reference to compare the results to the normal hatching percentage) were included. The follow-up of nematode eggs hatching was carried out at 7 and 14 days.

### Characterization of cell-free culture supernatants (CS) by HPLC analysis:

CS samples from B24 and DSM 8609 grown in TSB medium were analyzed by HPLC (eAlliance system, Waters, Milford, MA). Chromatograms were performed using an XBridge C18 reversed-phase column (5µm 4.6x150mm, Waters) eluted at 1 ml/min with a mobile phase mixture of solvents A [H₂O + 0.05% trifluoracetic acid (TFA)] and B (CH3CN + 0.05% TFA). The elution gradient was 0-10%B 10min, 10-40%B 10min, 40-100%B 3min, 100%B 2min, 0%B 5min. Produced metabolites were detected by UV spectroscopy with a photodiode array detector and chromatograms were obtained by processing at 254nm.

### Comparison of the cell-free culture supernatant enzymatic activities with other M. esteraromaticum strain supernatant:

The enzymatic activities from cell-free culture supernatants from the B24 *M. esteraromaticum* strain and the DSM 8609 type strain were evaluated using the API-ZYM^{®} method, as described previously in point1.2. The culture of the strains and the obtention of the cell-free culture supernatants were performed as described previously herein.

### Results:

Two weeks after applying the different treatments, 40.93% of the untreated eggs (control) and 39.22% of the treated eggs with TSB hatched. By contrast, only 29.74% and 27.25% of eggs hatched after treatment with CS of B24 grown in bioreactor and flask, respectively. Therefore, the efficacy of CS of B24 in reducing hatching with respect to the control was around 30% under *in vitro* conditions.

Root knot nematode eggs treated with CS produced by B24 showed a significant reduction on egg hatching with respect to the control with distilled water or TSB medium. The percentage of efficacy at 14 days after treatment, corrected with respect to the control (distilled water) was: 27.34% efficacy of CS from B24 obtained in bioreactor, in comparison with 4.17% efficacy of TBS medium; and 33.42% efficacy of CS from B24 (flask) compared with 3.46% efficacy of CS from DSM 8609 (flask). The efficacy of CS of the collection strain DSM 8609 grown at the same conditions of B24 was therefore lower. Therefore, the supernatant of the *M. esteraromaticum* strain B24 had nematicidal effect.

The different chromatographic profiles of B24 and DSM 8609 suggested that each strain produced distinct metabolites (see figure 9).

According to API-ZYM^{®}, the CS produced by B24 contained N-acetyl-β-glucosaminidase, leucine arylamidase, α-glucosidase and α-mannosidase. The CS produced by B24 did not contained Alkaline phosphatase, Esterase (C 4), Esterase Lipase (C 8), Lipase (C 14), Valine arylamidase, Cystine arylamidase, Trypsin, α-chymotrypsin, Acid phosphatase, Naphtol-AS-BI-phosphohydrolase, α-galactosidase, β galactosidase, β-glucuronidase, β-glucosidase, nor α-fucosidase.

B24 produced N-acetyl-β-glucosaminidase when it was grown in TSB medium, whereas in solid medium (AN), B24 did not produced this enzyme (as seen in point 1.2).

On the other hand, CS produced by DSM 8609 only showed leucine arylamidase activity. The CS produced by DSM 8609 did not contained N-acetyl-β-glucosaminidase, α-glucosidase and α-mannosidase, Alkaline phosphatase, Esterase (C 4), Esterase Lipase (C 8), Lipase (C 14), Valine arylamidase, Cystine arylamidase, Trypsin, α-chymotrypsin, Acid phosphatase, Naphtol-AS-BI-phosphohydrolase, α-galactosidase, β galactosidase, β-glucuronidase, β-glucosidase, nor α-fucosidase.

According to these results, it was demonstrated that B24 and DSM 8609 produced and liberated different metabolites to the medium. It was demonstrated that the supernatants of both strains were different.

### 1.7: Genetic comparison with other Microbacterium strains

### Materials and methods

The genome of the B24 strain of the present invention was compared to other Microbacterium species and *M. esteraromaticum* strains using ANIm described in Richter M, et al. JSpeciesWS: a web server for prokaryotic species circumscription based on pairwise genome comparison. Bioinformatics. 2015 Nov 16. pii: btv681. Among the Microbacterium species, a comparison was performed using certain strains of the DMSZ German Collection of Microorganisms and Cell Cultures, such as, *M. arborescens* DSM 20754 [T],, *M. azadirachtae* DSM 23848 [T], *M. foliorum* DSM 12966 [T], and *M*. *ginsengisoli* DSM 18659 [T]. The comparison with other M. *esteraromaticum* strains was performed using MM1 (GenBank accession number PDVO02) and B Mb 05.01 (GenBank accession number FUKO01) *M. esteraromaticum* strains.

### Results

The ANIm values obtained within the Microbacterium genera was of around 84, a relatively low value within the same genera according to Richter *et al.* (2015)

In relation to the *M. esteraromaticum* strains, the ANIm value of the B24 and MM1 was 87.71, the ANIm value of the B24 and B Mb 05.01 was 84.72, and the ANIm value of MM1 and B Mb 05.01 was 84.80.

### Example 2: In vitro nematicidal activity of Microbacterium esteraromaticum strain B24 against eggs of the RKNs

The *in vitro* nematicidal ability of bacterium *M. esteraromaticum* strain B24 on a mixture of RKN eggs was evaluated in comparison with a reference chemical (examples 2.1 and 2.2) or in comparison with other *M. esteraromaticum* strains (example 2.3) (one from the DSMZ collection and another one from the collection of Futureco Bioscience SA).

### Materials and methods

The tests were done in the dark to mimic soil conditions in an incubator (INCUBIG 288L 2000237, JP Selecta, Spain). The incubation temperature was 26°C.

The *in vitro* nematicidal ability of bacterium *M. esteraromaticum* strain B24 on a mixture of RKN eggs (*M. javanica* and *M. incognita*) (which was obtained from a tomato plant culture) was evaluated in hatching chambers (Nunclon^{™} Surface multiwell plates, Nunc, Denmark) in which 2 g of previously sterilized sand was added. The substrate was then inoculated with an aqueous suspension containing eggs of the previously mentioned RKNs: 120 eggs in example 2.1; 125 eggs in example 2.2; and 400 eggs in example 2.3. Immediately after, 200 µl of the biological control agent *M. esteraromaticum* strain B24 was applied at 8.0x10⁸ CFU/mL in example 2.1, at 3x10⁷ CFU/mL in example 2.2 and at 5.20x10⁷ CFU/mL in example 2.3.

In examples 2.1 and 2.2, a control with a reference chemical at the commercially recommended dose (Nemacur^{®} 0.0125%, active ingredient: Fenamiphos 24%, Bayer) and a control with sterile distilled water (to establish a reference to compare the results to the normal hatching percentage) were included.

In example 2.3, comparison was made with two other *M. esteraromaticum* strains: *M. esteraromaticum* DSMZ 8609 (applied at 2.03x10⁷ CFU/mL) and *M. esteraromaticum* strain B2538 (Futureco Bioscience Microorganisms Collection) (applied at 6.20x10⁷ CFU/mL).

The hatching chamber was incubated at 26°C for 3 weeks and periodic readings were done at 7, 14 and 21 days (in examples 2.1 and 2.2) or at 7 and 14 days (in example 2.3) after applying the biological control agent to determine the hatching percentage (the counts were performed by means of a Hawksley^{®} chamber; microscope OPTECH BIOSTAR B4, Optech Optical Technology, Germany). Six repetitions of each treatment were performed.

### Results:

### Example 2.1:

*M. javanica* and *M. incognita* eggs treated with *M. esteraromaticum* strain B24 showed a significant reduction on egg hatching with respect to the control (Figure 1).

Three weeks after applying the different treatments, 27.38% of the untreated eggs (control) hatched, whereas 5.37% of those treated with *M. esteraromaticum* strain B24 hatched and 2.50% of those treated with Nemacur^{®} (reference chemical) hatched. At day 21 after treatment *M. esteraromaticum* strain B24 reached 80.36% of efficacy in reducing hatching with respect to the control under *in vitro* conditions. The efficacy in reducing hatching was similar (there were no statistically significant differences) to the reference chemical (Nemacur^{®}), which had an efficacy in reducing hatching of 90.86%.

### Example 2.2:

*M. javanica* and *M. incognita* eggs treated with *M. esteraromaticum* strain B24 showed a significant reduction on hatching with respect to the control (Figure 2).

Three weeks after applying the different treatments, 35.89% of the untreated eggs (control) hatched, whereas 14.57% of those treated with M. *esteraromaticum* strain B24 hatched and 3.99% of those treated with Nemacur^{®} (reference chemical) hatched. Therefore, *M. esteraromaticum* strain B24 reached 59.40% of efficacy in reducing hatching with respect to the control under *in vitro* conditions, whereas in the reference chemical, efficacy in reducing hatching was 88.89%, i.e., higher than the reduction reached by the microorganism object of the present invention (*M*. *esteraromaticum* strain B24) but no statistical differences were observed between Nemacur^{®} and *M. esteraromaticum* strain B24 treatments.

### Example 2.3:

*M. javanica* and *M. incognita* eggs treated with *M. esteraromaticum* strain B24 showed a significant reduction on hatching with respect to the control, the strain B2538 and the reference strain DSMZ 8609 (Figure 3).

Two weeks after applying the different treatments, 38.46% of the untreated eggs (control) hatched, whereas 20.89% of those treated with *M. esteraromaticum* strain B24, 35.26% of those treated with *M. esteraromaticum* strain DSMZ 8609 and 32.23% of those treated with *M. esteraromaticum* strain B2538 hatched. Therefore, *M. esteraromaticum* strain B24 reached 45.69% of efficacy in reducing hatching with respect to the control under *in vitro* conditions, whereas the reference strain (DSMZ 8609) presented 8.32% of efficacy and the strain B2538 showed 16.20% of efficacy.

### Example 3: In vitro nematicidal activity of Microbacterium esteraromaticum strain B24 against juveniles (J2) of RKNs

The *in vitro* nematicidal ability of bacterium *M. esteraromaticum* strain B24 was evaluated against a mixture of the RKN *M. javanica* and *M. incognita* juveniles (J2).

### Materials and Methods:

The tests were done in the dark to mimic soil conditions in an incubator (INCUBIG 288L 2000237, JP Selecta, Spain). The incubation temperature was 26°C. The mixture of RKN *M. javanica* and M. *incognita* juveniles (infective juveniles stage, J2) proceeded form a culture in a tomato plant, and they were placed in hatching chambers (Nunclon^{™} Surface multiwell plates, Nunc, Denmark), in which 2 g of previously sterilized soil were added. Two experiments were performed:

### Example 3.1:

An aqueous suspension containing a mixture of 225 juvenile forms of *M. javanica + M. incognita* was delivered, and then 200 µL of the biological control agent, M. *esteraromaticum* strain B24 was applied at 1.40x10⁷ CFU/mL or at 2.10x10⁶ CFU/mL. Six repetitions of each treatment were performed.

### Example 3.2:

An aqueous suspension containing 275 juvenile forms of the mentioned nematode species (a mixture of them) was delivered, and then three different strains of *M. esteraromaticum* were applied at different doses: *M. esteraromaticum* strain B24 was applied at 5.20x10⁷ CFU/mL, *M. esteraromaticum* DSMZ 8609 was applied at 2.03x10⁷ CFU/mL and *M. esteraromaticum* strain B2538 was applied at 6.20x10⁷ CFU/mL. The experiment design consisted of three repetitions per treatment.

In both cases (examples 3.1 and 3.2), the hatching chamber was incubated at 26°C for 14 days and counts were performed by means of the Hawksley^{®} chamber after 7 days and 14 days to determine the number of live juveniles (%survival) (microscope OPTECH BIOSTAR B4, Optech Optical Technology, Germany). A negative control with sterile distilled water was included.

### Results:

### Example 3.1:

RKN juveniles treated with *M. esteraromaticum* strain B24 showed a significant reduction on the survival of the juveniles with respect the control when it was applied at the highest dose (1.40x10⁷ cfu/mL) (Figure 4).

Two weeks after applying the different treatments, 86.75% of the juveniles (negative control) survived, whereas70.46% of those treated with *M. esteraromaticum* strain B24 applied at 2.10x10⁶ CFU/mL survived and 21.39% of those treated with *M. esteraromaticum* strain B24 applied at 1.40x10⁷ survived. Therefore, *M. esteraromaticum* strain B24 reached 75.34% of efficacy in reducing the survival of juveniles with respect to the control under *in vitro* conditions when it was applied at a concentration of 1.40x10⁷CFU/mL, whereas when it was applied at a dose of 2.10x10⁶ CFU/mL, efficacy in reducing juveniles was about 18.78%. Therefore, there were statistical differences between the efficacy achieved by the microorganism object of the present invention (*M*. *esteraromaticum* strain B24) when it was applied at the highest dose (1.40x10⁷CFU/mL).

### Example 3.2:

Juveniles treated with *M. esteraromaticum* strain B24 showed a significant reduction on juveniles survival with respect to strain B2538 and the reference strain DSMZ 8609 (Figure 5).

Two weeks after applying the different treatments, 87.97% of the untreated juveniles (control) survived, whereas 61.42% of those treated with *M. esteraromaticum* strain B24 survived, 78.75% of those treated with *M. esteraromaticum* strain DSMZ 8609 and 81.17% of those treated with *M. esteraromaticum* strain B2538 survived. Therefore, *M. esteraromaticum* strain B24 reached 30.19% of efficacy in reducing juvenile survival with respect to the control under *in vitro* conditions, whereas the reference strain (DSMZ 8609) presented 10.49% of efficacy and the strain B2538 showed 7.73% of efficacy.

### Example 4: In vitro nematicidal activity of Microbacterium esteraromaticum strain B24 against eggs of Potato Cyst Nematodes

The *in vitro* nematicidal ability of bacterium *M. esteraromaticum* strain B24 on eggs of Potato Cyst Nematodes (PCNs) *Globodera rostochiensis* and *Globodera pallida* was evaluated.

### Materials and Methods

The tests were done in the dark to mimic soil conditions in an incubator (INCUBIG 288L 2000237, JP Selecta, Spain). The incubation temperature was 26°C. Hatching chambers (Nunclon^{™} Surface multiwell plates, Nunc, Denmark) in which 2 g of previously sterilized sand was added were used.

*Globodera* cysts were obtained from an infested soil using a Fenwick Can (flotation method). Cysts were disinfected with 2% bleach for 2 minutes and rinsed with sterile distilled water. Then cysts were then mechanically crushed in order to release *Globodera* eggs from inside. The *in vitro* test consisted of three treatments with 4 repetitions each: 1) Control eggs; 2) Eggs treated with *M. esteraromaticum* strain B24 (at 7x10⁷ CFU/mL)); and 3) Chemical control (Vydate^{®}, Oxamyl 24%, DuPont), applied at the commercial dose 0.2%.

Three hundred and twenty five eggs were placed in each well. Then, 4.5 mL of 10% Potato Root Diffusate (PRD) solution was added per well in order to induce eggs to hatch.

To prepare the PRD, Kennebec variety potato sprouts were seeded in trays containing sterile substrate (peat/vermiculite; 1/1, v/v). Sprouted potatoes were left in a climatic chamber for 3 weeks. Then roots of three-week-old plants were submerged in sterile distilled water for 24 hours at 25°C. After 24 hours, the distilled water that had been in contact with the roots was centrifuged (4000 rpm, 15 minutes), filtered (22µm) and stored in the freezer until needed.

Once the PRD was added, a solution of *M. esteraromaticum* strain B24 with a concentration of 7.70x10⁷ CFU/mL was added (200µL/well). The hatching chambers were maintained at 26°C throughout the evaluation period. Test readings were taken at 14, 28 and 35 days (using a Hawksley^{®} camber), and 1 mL of new 10% PRD was added every week.

### Results

Eggs treated with *M. esteraromaticum* strain B24 showed a significant reduction on hatching with respect to the control (Figure 6).

The percentage of efficacy 35 days after treatment with M. *esteraromaticum* strain B24 was 60.18%, corrected with respect to the control used as reference whereas in the chemical control the percentage of efficacy was 31.90%.

### Example 5: In vitro nematicidal activity of Microbacterium esteraromaticum strain B24 against cysts of PCNs

### Materials and Methods

The *in vitro* nematicidal ability of bacterium *M. esteraromaticum* strain B24 on cysts of PCN *Globodera rostochiensis* and *Globodera pallida* (a mixture of both species 50% each) was evaluated in hatching chambers (Nunclon^{™} Surface multiwell plates, Nunc, Denmark). The tests were done in the dark in an incubator (INCUBIG 288L 2000237, JP Selecta, Spain).

*Globodera* cysts were obtained from an infested soil using a Fenwick can (Flotation Method), then they were disinfected as described in example 4, but they were not mechanically crushed. The PRD was obtained as described in the previous bioassay (example 4)

### Example 5.1: A first in vitro test consisted of three treatments with 4 repetitions each:

1) Control cysts - untreated;
2) Control cysts treated with a chemical standard (Vydate^{®}, applied at the commercial dose 0.2%, Oxamyl 24%, DuPont); and
3) Cysts treated with *M. esteraromaticum* strain B24 as Technical Grade Active Ingredient (TGAI at 7x10⁷ CFU/mL) (TGAI diluted in water).

### Example 5.2: A second in vitro test consisted of three treatments with 4 repetitions each:

1) Control cysts - untreated;
2) Control cysts treated with a chemical standard (Vydate^{®}, Oxamyl 24%, applied at the commercial dose 0.2%, DuPont); and
3) Cysts treated with *M. esteraromaticum* strain B24 Formulation OD applied at 1%; Fomulation consisted of mixture of soy vegetable oil (Gustave Hess, Germany) 700g/L + Silica (Silysiamont, Italy) (10g/L)+ C18 ethoxilated fatty acid (Lamberti-Chemical Specialist, Italy) (200g/L) + polyacrylate crosspolymer-6 (Croda Europe Ltd. United Kingdom) (10g/L) + TGAI (strain of the invention) (80g/L). The materials were mixed in a laboratory reactor using a high speed stirrer designed for oils.

The strain of the invention in the TGAI was in a concentration of 1.2-1.3x10¹¹ CFU/g, there for, as used at 8% in the OD formulation, then in said formulation it was at 2.19x10¹⁰ CFU/mL.

Five cysts were placed in each well in both examples. Then, 4.5 mL of 10% PRD solution was added per well in order to induce eggs to hatch. Once the PRD was added, 200µL per well of a solution of M. *esteraromaticum* strain B24 with a concentration of 7.70x10⁷ CFU/mL was poured in example 5.1 and 200µL per well of a solution with *M. esteraromaticum* strain B24 Formulation OD applied at 1%; was poured in example 5.2. The hatching chambers were maintained at 26°C throughout the evaluation period. Test readings were taken at 14, 28 and 35 days (using a Hawksley^{®} chamber), and 1 mL of new 10% PRD was added every week.

### Results

Eggs treated with TGAI M. *esteraromaticum* strain B24 (Example 5.1.) or Formulation OD 1% of M. *esteraromaticum* strain B24 (Example 5.2.) showed a significant reduction on hatching with respect the control (Figures 7 and 8, respectively).

The percentage of efficacy after 35 days after treatment with TGAI *M. esteraromaticum* strain B24 was 77.92%, corrected with respect to the control used as reference whereas in the chemical control the percentage of efficacy was 35.72%.

The percentage of efficacy after 35 days after treatment with Formulation OD 1% of *M. esteraromaticum* strain B24 was 75.72%, corrected with respect to the control used as reference whereas in the chemical control the percentage of efficacy was 26.04%.

### Example 6: In vivo nematicidal activity of Microbacterium esteraromaticum strain B24 against RKNs

Three examples were performed using the *M. esteraromaticum* strain B24 in tomato plants: as "pellet (example 6.1), as TGAI (example 6.2) and as a formulated prototype (example 6.3). The results for all of them are presented at the end of this section. An example using the *M. esteraromaticum* strain B24 in cucumber plants is disclosed in example 6.4.

### Materials and Methods

### 6.1 In vivo nematicidal activity of a "pellet" of Microbacterium esteraromaticum strain B24

The *in vivo* nematicidal ability of bacterium *M. esteraromaticum* strain B24 was assessed in two experiments in climatic chamber: against *M. javanica + M. incognita.* The experimental design was composed by two different treatments: 1) Control with untreated plants; 2) plants treated with a suspension of *M. esteraromaticum* strain B24 applied as a "pellet". Each treatment consisted of 3 replicates with 3 plants per replicate.

The pellet was obtained as follows: 1mL of a frozen sample of the strain B24 (cryovial, -80°C) was thawed and grown in Tryptic soy broth (TSB) in a 100mL flask for 3 days approx. at 30 ° C. It was centrifuged and that pellet that remained (the microorganism as such) was tested in this assay.

### Example 6.1.1

Eighteen tomato seedlings ("Marmande" variety) 3-4 weeks old were transplanted into 1000 cm³ pots filled with a substrate which consisted of a mixture of sand and perlite (3:1; v:v). One day after transplant, all tomato plants were inoculated with a water suspension with root knot nematodes *M. javanica + M. incognita* (at a dose of 100 infective juveniles per 100 cm³ of substrate) (1000 juveniles/plant were the total addition of both nematodes, they were obtained from a tomato plant). Half of the plants were treated twice with 20 mL of an aqueous solution containing *M. esteraromaticum* strain B24, firstly 7 DAI (days after nematode inoculation) at a dose of 1.00x10⁷ CFU/mL and secondly 21 DAI at a dose of 1.03x10⁷ CFU/mL (in table 3 below, named as treatments A and B, respectively).

### Example 6.1.2

Eighteen tomato seedlings ("Marmande" variety) 3-4 weeks old were transplanted into 1000 cm³ pots filled with a substrate which consisted of a mixture of sand and perlite (3:1; v:v). Three days before transplant (preventive treatment), one third of the plants were treated with 10 mL of an aqueous solution containing *M. esteraromaticum* B24 at a dose of 2.13x10⁸ CFU/mL. One day after transplant, all tomato plants were inoculated with a water suspension with root knot nematodes, *M. javanica* (at a dose of 100 infective juveniles per 100 cm³ of substrate). Then, 20 mL of an aqueous solution containing *M. esteraromaticum* strain B24 was applied to one third of the plants 7 days after inoculation with the nematodes (7 DAI) at a dose of 4.9x10⁷ CFU/mL. In table 3 below, named as treatments A and B, respectively.

In both experiments (6.1.1 and 6.1.2) the plants were set up in a climatic chamber (temperature: 22±2°C; relative humidity (RH): 50±10 for 10 weeks since the seedlings were transplanted.

### Example 6.2: In vivo nematicidal activity of Microbacterium esteraromaticum strain B24 "TGAI" against RKNs

The *in vivo* nematicidal ability of bacterium *M. esteraromaticum* strain B24 was assessed in two experiments in greenhouse trials: example 6.2.1 and example 6.2.2, both against a mixture of M. *javanica* and *M. incognita* (obtained from a tomato plant).

The experimental design was composed by two different treatments: 1) Control with untreated plants; 2) Plants treated with a suspension of *M. esteraromaticum* strain B24 as TGAI (obtained from fermentation in bioreactor) at a dose in average of 7.88x10⁷ cfu/mL in example 6.2.1, and 1.55x10⁸ in average in example 6.2.2. The three treatments consisted of 3 replicates with 3 plants per replicate.

Eighteen tomato seedlings ("Durinta" variety) 3-4 weeks old were transplanted into 3000 cm³ pots filled with a substrate which consisted of a mixture of sand and (3:1; volume:volume). One day after transplant, all tomato plants were inoculated with a water suspension with RKN *Meloidogyne javanica + Meloidogyne incognita* (at a dose of 100 infective juveniles per 100 cm³ of substrate) (it was a mixture of said nematodes, 50% of *Meloidogyne javanica* (population of code "AL05" from Futureco Bioscience) plus 50% of *Meloidogyne incognita* (population of code "AL09" from Futureco Bioscience), the two populations were isolated in Almeria, Spain).

The half of the plants were treated once with 10 mL of an aqueous solution containing *M. esteraromaticum* strain B24 at a particular dose indicated in the table 1 below three days before transplant (preventive treatment). Then, 20 mL of an aqueous solution containing *M. esteraromaticum* strain B24 were applied to the same plants 7 days after inoculation with the nematodes (7 DAI), at 21 DAI and at 35 DAI, at the doses indicated in the table 1. Experiment 6.2.1 and 6.2.2 received different doses.

**Table 1. M. esteraromaticum strain B24 application plant in an in vivo greenhouse trial against M. javanica and Meloidogyne incognita in tomato plants. In Table 3 below, those treatments are called A, B, C and D.**

| | **Day 0** | **Day 3** | **Day 4** | **Day 11** | **Day 25** | **Day 39** |
|---|---|---|---|---|---|---|
| Experiment | 1st Application (A, preventive) | Transplant | Nematode inoculation | 2^{nd} Application (B, curative) | 3^{nd} Application (C, curative) | 4^{th} Application (D, curative) |
| 6.2.1 | 6.2x10⁷ CFU/mL | | | 1.50x10⁷ CFU/mL | 1.70x10⁸ CFU/mL | 1.31x10⁸ CFU/mL |
| 6.2.2 | 2.8x10⁸ CFU/mL | | | 5.0x10⁷ CFU/mL | 1.20x10⁸ CFU/mL | 1.68x10⁸ CFU/mL |

In both experiments (6.2.1 and 6.2.2) the plants were set up in greenhouse (temperature: 25±2°C; relative humidity (RH: 50±10) for 9 weeks since the seedlings were transplanted.

### Example 6.3: In vivo nematicidal activity of Microbacterium esteraromaticum strain B24 "Formulated Prototypes" against RKNs

The *in vivo* nematicidal ability of a nematicidal formulation based on the bacterium *M. esteraromaticum* strain B24 was assessed in two experiments in greenhouse trials in tomato plants (example 6.3.1 and example 6.3.2, both against a mixture of *M. javanica* and *M. incognita* (obtained from a tomato plant).

The experimental design was composed by two different treatments: 1) Control with untreated plants; 2) Plants treated with a suspension of bacterium *M. esteraromaticum* strain B24 applied as a "formulation" at a dose in average 6.43x10⁷ cfu/mL (Formulation-10⁷) in example 6.3.1 or at a dose in average 2.30x10⁷ CFU/mL in example 6.3.2 (Formulation-10⁷). Both treatments consisted of 3 replicates with 3 plants per replicate. Fomulation consisted of mixture of: soy vegetable oil (Gustave Hess, Germany) 700g/L + Silica (Silysiamont, Italy) (10g/L)+ C18 ethoxilated fatty acid (Lamberti-Chemical Specialist, Italy) (200g/L) + polyacrylate crosspolymer-6 (Croda Europe Ltd. United Kingdom) (10g/L) + TGAI (strain of the invention) (80g/L) (the strain of the invention in the TGAI was in a concentration of 1.2-1.3x10¹¹ CFU/g, there for, as used at 8% in the OD formulation, then in said formulation it was at 1x10¹⁰ CFU/mL). The materials were mixed in a laboratory reactor using a high speed stirrer designed for oils.

Eighteen tomato seedlings ("Durinta" variety) 3-4 weeks old were transplanted into 3000 cm³ pots filled with a substrate which consisted of a mixture of sand and perlite (3:1; volume:volume). One day after transplant, all tomato plants were inoculated with a water suspension with root knot nematodes, *Meloidogyne javanica+M.incognita* (at a dose of approximately 100 infective juveniles (it was a mixture of the two nematodes) per 100 cm³ of substrate). There were two different sets of plants: Half of the plants were untreated (control plants). The other half of the plants was treated once with 20mL of an aqueous solution containing 1% of a formulation based on *M. esteraromaticum* strain B24 at a dose indicated in the table 2 below at the day of transplant (preventive treatment). Then, 20mL of an aqueous solution containing 1% of the formulation based on *M. esteraromaticum* strain B24 were applied to the same half of the plants 7 days after inoculation with the nematodes (7 DAI), at 21 DAI and at 35 DAI, at the doses indicated in the table 2 below for each experiment.

**Table 2. Formulation based on M. esteraromaticum strain B24 application plant in vivo greenhouse trial against RKN in tomato plants. In Table 4 below, those treatments are called A, B, C and D.**

| | **Day 1** | **Day 2** | **Day 9** | **Day 23** | **Day 37** |
|---|---|---|---|---|---|
| Example | Transplant + 1st Application (A, preventive) | Nematode inoculation | 2^{nd} Application (B, curative) | 3^{nd} Application (C, curative) | 4^{th} Application (D, curative) |
| 6.3.1 | 2.50x10⁸ CFU/mL | | 2.00x10⁵ CFU/mL | 1.90x10⁶ CFU/mL | 5.20x10⁶ CFU/mL |
| 6.3.2 | 2.30x10⁷ CFU/mL | | 2.00x10⁷ CFU/mL | 2.40x10⁷ CFU/mL | 2.50x10⁷ CFU/mL |

In both experiments (6.3.1 and 6.3.2) the plants were set up in greenhouse (temperature: 25±2°C; relative humidity (RH): 50±10) for 9 weeks since the seedlings were transplanted.

For all experiments (experiments 6.1, 6.2 and 6.3), at the end of the bioassay the total number of eggs per plant and eggs per gram of root were determined in each treatment: they were extracted from the radicular system by mechanical disruption and passed through different sieves from 200 mesh (75 µm) to 500 mesh (25 µm). Eggs were visualized in Hawksley^{®} chamber; microscope OPTECH BIOSTAR B4, Optech Optical Technology, Germany.

Pf in means of number of eggs/plant and nematode Reproduction (eggs/gram of fresh root) was assessed in all treatments 10 or 9 weeks after transplant (10 weeks in the case of the "pellet" and 9 weeks in the case of the TGAI and OD formulation).

In all cases after the treatments performed, data were subjected to analysis of variance (ANOVA) using R software (R Core Team, 2013, Austria). The significant differences among treatments were determined by the Least Significant Difference (LSD) test at P≤0.05.

### Results of examples 6.1 ("pellet"), 6.2 (TGAI) and 6.3 (formulated prototype)

The RKN treated with M. *esteraromaticum* strain B24 in the three formulations showed a significant reduction on both parameters with respect to the control in all cases (see table 3 below). The efficacy on Reproduction was more similar between treatments as the strain was given in every case per gram of root, whereas the efficacy on Final Population was measured by plant (where differences between development of root system can make differences between repetitions). The results obtained in this assay showed that a formulation based on *M. esteraromaticum* strain B24 applied as a pellet, as a TGAI or at a 1% OD dose had nematicidal activity against RKN.

**Table 3: Efficacy (%) of M. esteraromaticum strain B24 (applied as a ·pellet", "TGAI" or "Formulation" against RKN in tomato plants in greenhouse corrected with respect to the control used as a reference.**

| **Treatments** | **CFU/mL** | **Efficacy (%) on Final Population (P_{f})(eggs/plant)** | **Efficacy (%) on Reproduction (eggs/g root)** |
|---|---|---|---|
| *M. esteraromaticum* strain B24 "pellet" (Example 6.1.1.) | A: 1.00x10⁷ | 72.50 | 68.53 |
| | B: 1.03x10⁷ | | |
| *M. esteraromaticum* strain B24 "pellet" (Example 6.1.2.) | A: 2.13x10⁸ | 39.04 | 54.50 |
| | B: 4.90x10⁷ | | |
| *M. esteraromaticum* strain B24 "TGAI" (Example 6.2.1.) | A: 6.20x10⁷ | 34.77 | 56.30 |
| | B: 1.50x10⁷ | | |
| | C: 1.70x10⁸ | | |
| | D: 1.31x10⁸ | | |
| *M. esteraromaticum* strain B24 "TGAI" (Example 6.2.2.) | A: 2.80x10⁸ | 46.04 | 46.89 |
| | B: 5.00x10⁷ | | |
| | C: 1.20x10⁸ | | |
| | D: 1.68x10⁸ | | |
| *M. esteraromaticum* strain B24 - Formulation (Example 6.3.1) | A: 2.50x10⁸ | 51.28 | 42.46 |
| | B: 2.00x10⁵ | | |
| | C: 1.90x10⁶ | | |
| | D: 5.20x10⁶ | | |
| *M. esteraromaticum* strain B24 - Formulation (Example 6.3.2.) | A: 2.30x10⁷ | 57.59 | 56.83 |
| | B: 2.00x10⁷ | | |
| | C: 2.40x10⁷ | | |
| | D:2.50x10⁷ | | |

### Example 6.4: In vivo nematicidal activity of Microbacterium esteraromaticum strain B24 against RKNs in cucumber plants

### Materials and Methods

The *in vivo* nematicidal ability of a nematicidal formulation based on the bacterium *M. esteraromaticum* in cucumber plants was assessed. Similar material and methods as the ones used in examples 6.3 were used, with the exception that cucumber of "Dasher" variety were used, 6 plants per treatment were used (2 plants per block and 3 replicates) and at the doses indicated in the table 4 below. Chemical standard SONDAE (active ingredient Oxamyl 10%, SAPEC AGRO) was used (see table 5 below).

**Table 4. Formulation based on M. esteraromaticum strain B24 application plant in vivo greenhouse trial against RKN in cucumber plants. In table 5 below, those treatments are called A, B, C and D.**

| | **Day 1** | **Day 2** | **Day 9** | **Day 23** | **Day 37** |
|---|---|---|---|---|---|
| Treatment | Transplant + 1st Application (A preventive) | Nematode inoculation | 2^{nd} Application (B curative) | 3^{nd} Application (C curative) | 4^{th} Application (D curative) |
| *M. esteraromaticum* strain B24 "TGAI" (10⁸ CFU/mL.) | 7.50 10⁷ CFU/mL | | 7.50 10⁷ CFU/mL | 7.80 10⁷ CFU/mL | 2.00 10⁷ CFU/mL |
| *M. esteraromaticum* strain B24 "OD" 2% | 3.8 10⁸ CFU/mL | | 3.0 108 CFU/mL | | |
| *M. esteraromaticum* strain B24 "OD" 1% | 2.53 10⁸ CFU/mL | | 2.19 10⁸ CFU/mL | 1.50 10⁸ CFU/mL | 2.00 10⁸ CFU/mL |
| *M. esteraromaticum* strain B24 "OD" 0.5% | 1.46 10⁸ CFU/mL | | 1.04 10⁸ CFU/mL | 9.70 10⁷ CFU/mL | 1.04 10⁸ CFU/mL |

### Results

*In vivo* experiments performed in cucumber plants showed good efficacy on final population and efficacy on Reproduction, the RKN treated with *M. esteraromaticum* strain B24 showed a significant reduction on both parameters with respect to the control (see table 5 below).

**Table 5. Efficacy (%) of M. esteraromaticum strain B24 (applied as "TGAI" or "Formulation") against RKN in cucumber var. "Dasher" plants in greenhouse corrected with respect to the control used as a reference. ANOVA, LSD, P <0.05.**

| **Treatments** | **CFU/mL** | **Efficacy (%) on Final Population (P_{f})(eggs/plant)** | **Efficacy (%) on Reproduction (eggs/g root)** |
|---|---|---|---|
| *M*. *esteraromaticum* strain B24 "TGAI" (10⁸ CFU/mL.) | A: 7.50 10⁷ | 73.96 a | 76.92 a |
| | B: 7.50 10⁷ | | |
| | C: 7.80 10⁷ | | |
| | D: 2.00 10⁷ | | |
| *M. esteraromaticum* strain B24 "OD" 2% | A: 3.8.13x10⁸ | 68.48 ab | 71.98 a |
| | B: 3.0 x10⁸ | | |
| *M. esteraromaticum* strain B24 "OD" 1% | A: 2.53x10⁸ | 68.30 ab | 73.78 a |
| | B: 2.19x10⁸ | | |
| | C: 1.50x10⁸ | | |
| | D: 2.00x10⁸ | | |
| *M. esteraromaticum* strain B24 "OD" 0.5% | A: 1.46x10⁸ | 58.13 bc | 64.24 b |
| | B: 1.04x10⁸ | | |
| | C: 9.70x10⁷ | | |
| | D: 1.04x10⁸ | | |
| Chemical standard (SONDAE, active ingredient: Oxamyl 10%) | B: 0.75% | 44.63 c | 46.21 b |

### Example7: Evaluation of oral toxicity of M. esteraromaticum strain B24

To determine whether the strain *M. esteraromaticum* B24 was safe, its oral toxicity in mice was tested.

### Materials and Methods:

Three male and three female Balb/C mice of 4-5 weeks old (QC'd albino pathogen free mice; Envigo) were used. Animals fasted overnight before dosing. The day before the experiment, the body weight of the animals was recorded and feces samples were collected from individual animals. Each animal received one oral single dose of the *M. esteraromaticum* B24 strain: 10⁹ CFU B24 TGAI (vehicle 200µl of sterile H₂O) (prepared as described previously in example 5.1). Mice that received sterile water (vehicle control) were also maintained throughout the study. Mice were housed under controlled environment and monitored for 21 days and the observations were recorded. Daily, once, the following was observed for each animal: skin and fur, eyes and mucous membranes, respiratory system, circulatory system, autonomic and central nervous system, somatomotor activity, behavior pattern, observation of tremors, convulsions, diarrhea, lethargy, salivation, and coma if any. Weights of individual animals checked prior and post gavage once per week. Necropsy was performed at the end of 21 day post oral gavage for assessment of presence or absence of the bacterial strain in the tissues. Tissue collection and blood culture was done aseptically to avoid any cross contamination.

Necropsy: the kidney, brain, liver, lung, spleen, blood and gut lymph node was collected from each animal and immediately kept in dry ice and stored at -20°C until further processing. RNA was extracted from the above tissues and RT-PCR was performed to determine presence of B24 TGAI (16S rRNA) using the specific primers: "M estera F1", (SEQ ID NO: 4); "M estera R1", (SEQ ID NO: 5); "M estera F2", (SEQ ID NO: 6); and "M estera R2" (SEQ ID NO: 7) (the two primer pairs were used in order to be sure of the specificity of the amplification; R, reverse primer; F, forward primer).

Blood Processing: at necropsy, aseptic whole blood samples were collected from each animal. Immediately following collection, 100µl of each blood sample was placed and spread on agar plate and maintained at 28 °C for 120 hours. The plates were analyzed at regular intervals for assessment of any bacterial growth.

Clearance of the Microbial Pest Control Agent (MPCA): feces collection on dosing and on day 7, 14 and 21 days were kept at 4 °C for further RNA extraction and RT-PCR analysis.

Blood/Bacterial culture media: Meat extract 1g, Yeast Extract 2g, Peptone 5g, NaCl 5g and Agar 15g were added (for 1000 ml) and sterilized for 121 °C for 20 min and thereafter was poured into plates and used for the blood culture and B24 TGAI CFU/g determination. RNA extraction protocol: 50 mg of tissue was used for RNA extraction following manufacturer's instructions (RNA Isolation kit Zymo Research, Cat No R1050).

Spike control: 100µl of 10⁹ CFU of B24 TGAI was spiked with 50mg of brain and liver tissue and total RNA was extracted by using the Quick RNA kit total RNA extraction kit according to manufacturer's instructions (Zymo Research) with addition of Proteinase K and Lysozyme digestion step -included in the aforementioned kit- for isolating total RNA including bacterial RNA.

Positive control: 0.2g of B24 TGAI diluted serially for 7 times in sterile water and plated (100 µl) and reconfirmed the CFU/g sample. The bacterial colonies grown on agar plates were collected and directly used for total RNA extraction and as a positive control for the study.

cDNA synthesis: Total RNA from tissues and feces were measured in a micro plate reader (Biotek). 2µg of total RNA (1µg in case of feces samples) was used for cDNA synthesis by using Superscript IV 1st strand synthesis kit (Life technologies, Cat No: 18091050). Appropriate positive (B24 TGAI) and negative controls (H₂O) were also maintained.

RT-PCR assay: 20µl total of the cDNA mixture was diluted 1:10 fold (tissue samples) and 1:5 fold (feces samples cDNA) and RT-PCR was performed using PowerUp SYBR^{™} Green Master Mix according to manufacturer's instructions (Life Technologies, Cat No A25776) with an Applied Biosystems StepOne^{™} real time PCR device. PCR reactions consisted of 5 µL PowerUp^{™} SYBR^{™} Green Master Mix, 0.5 µL each specific primer (F1 and R1, or F2 and R2), and 10 ng cDNA as template, in a total reaction volume of 20 µL. The thermal cycling conditions were as follows: 2 min at 50 °C, 2 min at 95 °C, and 40 cycles of 15 s at 95 °C, 15 s at 60 °C, and 1 min at 72 °C.

### Results:

Body weight from the control and *M. esteraromaticum* B24 gavaged animal were not significantly different. Blood culture plates incubated for 120h at 28 degree had no visible growth and was concluded negative. Clinical symptoms monitored did not reveal adverse changes. RT-PCR data revealed that the 16srDNA specific primers were not amplified in any of the tissue or feces samples from *M. esteraromaticum* B24 gavaged animals (Average Ct values 33). The *M. esteraromaticum* B24 TGAI spiked tissue samples were amplified using the *M esteraromaticum* specific primers along with the positive control (average spiked Ct value 12 for brain and 17 for liver). Negative controls (water gavaged) had no amplification in RT-PCR (Average Ct value 30). Positive control 16s rDNA primer amplified at cycle 6 (Ct value 6). The data revealed absence of *M esteraromaticum* B24 in any of the samples screened using the specific RT-PCR primers.

Animals had no visible clinical adverse effects and no infectivity or pathogenicity was found. *M. esteraromaticum* B24 strain gavaged animals had no visible clinical adverse effects and the bacterial strain was not detected in the blood, feces or other organs examined.

### REFERENCES CITED IN THE APPLICATION:

Sturz AV and Kimpinski J "Endoroot bacteria derived from marigolds (Tagetes spp.) can decrease soil population densities of root-lesion nematodes in the potato root zone" 2004 Plan and Soil 262:241-249.
Sambrook J and Russell DW "Molecular Cloning: A Laboratory Manual" Chapter 13 "Mutagenesis" Cold Spring Harbor, 3rd Ed, 2001.
Schwyn B and Neilands J B "Universal chemical assay for the detection and determination of siderophores." 1987 Anal Biochem 160(1): 47-56.
Altschul et al., "Basic local alignment search tool", 1990, J. Mol. Biol, v. 215, pages 403-410.
Higgins et al., "CLUSTAL V: improved software for multiple sequence alignment", 1992, CABIOS, 8(2), pages 189-191.
Richter M, et al. JSpeciesWS: a web server for prokaryotic species circumscription based on pairwise genome comparison. Bioinformatics. 2015 Nov 16. pii: btv681.
Aubert et al., "A Markerless Deletion Method for Genetic Manipulation of Burkholderia cenocepacia and Other Multidrug-Resistant Gram-Negative Bacteria" Methods Mol Biol 2014;1197:311-27.

## Claims

1. A strain of *Microbacterium esteraromaticum* deposited at the "Colección Española de Cultivos Tipo" (CECT) under the accession number CECT9167.

2. A bacterial culture comprising the strain according to claim 1, preferably the bacterial culture is an inoculation product.

3. A composition comprising an effective amount of the strain CECT9167 of *Microbacterium esteraromaticum* according to claim 1, or the bacterial culture according to claim 2, and one or more agriculturally acceptable compounds.

4. The composition according to claim 3 wherein the strain is present at a concentration from 10⁵ CFU/ml to 10¹² CFU/ml.

5. The composition according to any one of claims 3 or 4 wherein the agriculturally acceptable compound is selected from the group consisting of: plant strengtheners, nutrients, wetting agents, compounds that improve adherence, buffering compounds, stabilizers, antioxidants, osmotic protectors and sunscreens.

6. The composition according to any one of claims 3 to 5, which comprises at least one additional pesticide.

7. The composition according to any one of claims 3 to 6 wherein the composition comprises an oily ingredient, preferably a vegetable oil, preferably soy oil.

8. The composition according to any one of claims 3 to 7 wherein the composition comprises soy oil, C18 ethoxilated fatty acid, silica and polyacrylate crosspolymer.

9. A process for obtaining a viable cell suspension derived from the strain CECT9167 of *Microbacterium esteraromaticum* according to claim 1, the process comprising: (i) inoculating the strain in a suitable culture medium, (ii) subjecting the inoculated culture medium of the step (i) to conditions suitable for growth of the strain, and (iii) optionally subjecting the medium resulting from step (ii) to a concentration step.

10. A supernatant of the strain CECT9167 of *M. esteraromaticum* obtainable by a process comprising: (i) inoculating the strain CECT9167 of *Microbacterium esteraromaticum* according to claim 1 in a suitable culture medium; (ii) subjecting the inoculated culture medium to suitable growth conditions; (iii) separating the cells from the culture medium of step (ii); (iv) collecting the supernatant; and (v) optionally subjecting the supernatant to a concentration step.

11. A kit comprising an effective amount of the strain CECT9167 of *M. esteraromaticum* according to claim 1, the bacterial culture according to claim 2, the composition according to any one of claims 3 to 8 or the supernatant according to claim 10.

12. Use of the strain CECT9167 of *Microbacterium esteraromaticum* according to claim 1, or of the bacterial culture according to claim 2, or of the composition according to any one of the claims 3-8, or of the supernatant according to claim 10, or of the kit according to claim 11 for controlling a nematode infection in a plant.

13. A method for controlling an infection caused by nematodes in a plant comprising applying to a part of a plant or to the substrate used for growing said plant the strain CECT9167 of *Microbacterium esteraromaticum* according to claim 1, or the bacterial culture according to claim 2, or the composition according to any one of the claims 3-8, or the supernatant according to claim 10, or the kit according to claim 11.

14. The use according to claim 12 or the method according to claim 13, wherein: the nematode is selected from the genus *Meloidogyne* and *Globodera;* or alternatively, it is selected from the group consisting of *Meloidogyne incognita, Meloidogyne javanica, Globodera rostochiensis* and *Globodera pallida.*

## Patentansprüche

1. Ein Stamm von *Microbacterium esteraromaticum,* hinterlegt bei der "Colecciön Española de Cultivos Tipo" (CECT) unter der Hinterlegungsnummer CECT9167.

2. Eine Bakterienkultur umfassend den Stamm nach Anspruch 1, wobei die Bakterienkultur vorzugsweise ein Inokulationsprodukt ist.

3. Eine Zusammensetzung umfassend eine wirksame Menge des Stammes CECT9167 von *Microbacterium esteraromaticum* nach Anspruch 1 oder die Bakterienkultur nach Anspruch 2 und eine oder mehrere landwirtschaftlich akzeptable Verbindungen.

4. Die Zusammensetzung nach Anspruch 3, wobei der Stamm in einer Konzentration von 10⁵ KbE/ml bis 10¹² KbE/ml vorliegt.

5. Die Zusammensetzung nach einem der Ansprüche 3 oder 4, wobei die landwirtschaftlich akzeptable Verbindung ausgewählt ist aus der Gruppe bestehend aus: Pflanzenverstärkern, Nährstoffen, Netzmitteln, Verbindungen, die die Haftung verbessern, Pufferverbindungen, Stabilisatoren, Antioxidationsmitteln, osmotischen Schutzmitteln und Sonnenschutzmitteln.

6. Die Zusammensetzung nach einem der Ansprüche 3 bis 5, welche mindestens ein zusätzliches Pestizid umfasst.

7. Die Zusammensetzung nach einem der Ansprüche 3 bis 6, wobei die Zusammensetzung einen öligen Bestandteil, vorzugsweise ein Pflanzenöl, vorzugsweise Sojaöl, umfasst.

8. Die Zusammensetzung nach einem der Ansprüche 3 bis 7, wobei die Zusammensetzung Sojaöl, ethoxilierte C18-Fettsäure, Siliciumdioxid und Polyacrylat-Crosspolymer umfasst.

9. Ein Verfahren zum Erhalten einer lebensfähigen Zellsuspension, die von dem Stamm CECT9167 von *Microbacterium esteraromaticum* nach Anspruch 1 abgeleitet ist, wobei das Verfahren Folgendes umfasst: (i) Inokulieren des Stamms in einem geeigneten Kulturmedium, (ii) Unterziehen des inokulierten Kulturmediums aus Schritt (i) Bedingungen, die für das Wachstum des Stamms geeignet sind, und (iii) wahlweise Unterziehen des aus Schritt (ii) resultierenden Mediums einem Konzentrierungsschritt.

10. Ein Überstand des Stammes CECT9167 von *M. esteraromaticum,* der durch ein Verfahren erhalten werden kann, das Folgendes umfasst: (i) Inokulieren des Stamms CECT9167 von *Microbacterium esteraromaticum* nach Anspruch 1 in einem geeigneten Kulturmedium; (ii) Unterziehen des inokulierten Kulturmediums geeigneten Wachstumsbedingungen; (iii) Abtrennen der Zellen aus dem Kulturmedium aus Schritt (ii); (iv) Sammeln des Überstands; und (v) wahlweise Unterziehen des Überstands einem Konzentrierungsschritt.

11. Ein Kit umfassend eine wirksame Menge des Stammes CECT9167 von M. *esteraromaticum* nach Anspruch 1, die Bakterienkultur nach Anspruch 2, die Zusammensetzung nach einem der Ansprüche 3 bis 8 oder der Überstand nach Anspruch 10.

12. Verwendung des Stammes CECT9167 von *Microbacterium esteraromaticum* nach Anspruch 1 oder der Bakterienkultur nach Anspruch 2 oder der Zusammensetzung nach einem der Ansprüche 3 bis 8 oder des Überstands nach Anspruch 10 oder des Kits nach Anspruch 11 zur Bekämpfung einer Nematodeninfektion in einer Pflanze.

13. Ein Verfahren zur Bekämpfung einer durch Nematoden verursachten Infektion in einer Pflanze, umfassend das Aufbringen des Stamms CECT9167 von *Microbacterium esteraromaticum* nach Anspruch 1 oder der Bakterienkultur nach Anspruch 2 oder der Zusammensetzung nach einem der Ansprüche 3 bis 8 oder des Überstands nach Anspruch 10 oder des Kits nach Anspruch 11 auf einen Teil einer Pflanze oder auf das zum Züchten der Pflanze verwendete Substrat.

14. Die Verwendung nach Anspruch 12 oder das Verfahren nach Anspruch 13, wobei: der Nematode ausgewählt ist aus der Gattung *Meloidogyne* und *Globodera;* oder alternativ ausgewählt ist aus der Gruppe bestehend aus *Meloidogyne incognita, Meloidogyne javanica, Globodera rostochiensis* und *Globodera pallida.*

## Revendications

1. Une souche de *Microbacterium esteraromaticum* déposée à la « Colección Española de Cultivos Tipo » (CECT) sous le numéro de dépôt CECT9167.

2. Une culture bactérienne comprenant la souche selon la revendication 1, de préférence la culture bactérienne est un produit d'inoculation.

3. Une composition comprenant une quantité efficace de la souche CECT9167 de *Microbacterium esteraromaticum* selon la revendication 1, ou la culture bactérienne selon la revendication 2, et un ou plusieurs composés acceptables en agriculture.

4. La composition selon la revendication 3, dans laquelle la souche est présente à une concentration de 10⁵ UFC/ml à 10¹² UFC/ml.

5. La composition selon l'une quelconque des revendications 3 ou 4, dans laquelle le composé acceptable en agriculture est choisi dans le groupe constitué par : les renforçateurs de plantes, les nutriments, les agents mouillants, les composés qui améliorent l'adhérence, les composés tampons, les stabilisants, les antioxydants, les protecteurs osmotiques et les écrans solaires.

6. La composition selon l'une quelconque des revendications 3 à 5, qui comprend au moins un pesticide supplémentaire.

7. La composition selon l'une quelconque des revendications 3 à 6, dans laquelle la composition comprend un ingrédient huileux, de préférence une huile végétale, de préférence de l'huile de soja.

8. La composition selon l'une quelconque des revendications 3 à 7, dans laquelle la composition comprend de l'huile de soja, un acide gras éthoxylé en C18, de la silice et un polymère croisé de polyacrylate.

9. Un procédé pour obtenir une suspension cellulaire viable dérivée de la souche CECT9167 de *Microbacterium esteraromaticum* selon la revendication 1, le procédé comprenant : (i) inoculer la souche dans un milieu de culture approprié, (ii) soumettre le milieu de culture inoculé de l'étape (i) à des conditions appropriées pour la croissance de la souche, et (iii) facultativement soumettre le milieu résultant de l'étape (ii) à une étape de concentration.

10. Un surnageant de la souche CECT9167 de *M. esteraromaticum* pouvant être obtenu par un procédé comprenant : (i) inoculer la souche CECT9167 de *Microbacterium esteraromaticum* selon la revendication 1 dans un milieu de culture approprié ; (ii) soumettre le milieu de culture inoculé à des conditions de croissance appropriées ; (iii) séparer les cellules du milieu de culture de l'étape (ii) ; (iv) collecter le surnageant ; et (v) facultativement soumettre le surnageant à une étape de concentration.

11. Un kit comprenant une quantité efficace de la souche CECT9167 de M. *esteraromaticum* selon la revendication 1, la culture bactérienne selon la revendication 2, la composition selon l'une quelconque des revendications 3 à 8 ou le surnageant selon la revendication 10.

12. Utilisation de la souche CECT9167 de *Microbacterium esteraromaticum* selon la revendication 1, ou de la culture bactérienne selon la revendication 2, ou de la composition selon l'une quelconque des revendications 3 à 8, ou du surnageant selon la revendication 10, ou du kit selon la revendication 11 pour lutter contre une infection nématodique chez une plante.

13. Un procédé pour lutter contre une infection causée par des nématodes dans une plante, comprenant appliquer à une partie d'une plante ou au substrat utilisé pour la culture de ladite plante la souche CECT9167 de *Microbacterium esteraromaticum* selon la revendication 1, ou la culture bactérienne selon la revendication 2, ou la composition selon l'une quelconque des revendications 3 à 8, ou le surnageant selon la revendication 10, ou le kit selon la revendication 11.

14. L'utilisation selon la revendication 12 ou le procédé selon la revendication 13, où : le nématode est choisi dans le genre *Meloidogyne* et *Globodera ;* ou, en variante, il est choisi dans le groupe constitué par *Meloidogyne incognita, Meloidogyne javanica, Globodera rostochiensis* et *Globodera pallida.*
